(19) Europäisches Patentamt / European Patent Office / Office européen des brevets

(11) **EP 4 279 531 B1**

(12) **EUROPEAN PATENT SPECIFICATION**

(45) Date of publication and mention of the grant of the patent:
**25.02.2026 Bulletin 2026/09**

(21) Application number: **23171772.9**

(22) Date of filing: **05.05.2023**

(51) International Patent Classification (IPC):
*C08G 63/60* (2006.01)    *C08G 65/48* (2006.01)
*C08G 63/668* (2006.01)    *A61Q 19/00* (2006.01)

(52) Cooperative Patent Classification (CPC):
**C08G 63/668; A61K 8/35; A61K 8/37;
A61K 8/4966; A61K 8/86; A61Q 17/04; C08G 65/48**

(54) **CROSSLINKED POLYGLYCEROL ESTERS**

VERNETZTE POLYGLYCERINESTER

ESTERS DE POLYGLYCÉROL RÉTICULÉS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **18.05.2022 EP 22174044**

(43) Date of publication of application:
**22.11.2023 Bulletin 2023/47**

(73) Proprietor: **Evonik Operations GmbH
45128 Essen (DE)**

(72) Inventors:
 • **Hartung, Christian**
 **45133 Essen (DE)**
 • **von Hof, Jan Marian**
 **44789 Bochum (DE)**
 • **Mentel, Matthias**
 **44357 Dortmund (DE)**
 • **Friedrich, Achim**
 **45529 Hattingen (DE)**
 • **Kleinen, Jochen**
 **52525 Heinsberg (DE)**
 • **Venzmer, Joachim**
 **45239 Essen (DE)**
 • **Meyer, Juergen**
 **45259 Essen (DE)**
 • **Wenk, Hans Henning**
 **45470 Mülheim an der Ruhr (DE)**
 • **Klare, Sven**
 **51069 Köln (DE)**

(74) Representative: **Evonik Patent Association
c/o Evonik Industries AG
IP Management
Postcode 84/339
Rodenbacher Chaussee 4
63457 Hanau (DE)**

(56) References cited:
**EP-A2- 2 363 387    EP-B1- 1 683 781**

**Description**

Field of the invention

The invention provides polyglycerol partial esters based on mono- and dicarboxylic acids, the production and use thereof, in cosmetics in particular.

Prior art

Commercially available cosmetic film formers for sun protection and make-up formulations are mostly high-molecular-weight synthetic/petrochemically based, non-biodegradable polymers such as polyurethanes, polyacrylates, polyolefins, polyvinylpyrrolidones or the corresponding copolymers thereof. Examples thereof are products with the Antaron/Ganex (Ashland), Baycusan (Covestro) or Tego SP (Evonik) trade names.

Reasonably hydrophilic and thus reasonably readily water-soluble/water-swellable film formers based on natural polymers have likewise been described and are based for example on polysaccharides such as starch, cellulose and derivatives thereof or polypeptides. However, these do not exhibit good water resistance and often also show inadequate sun protection factor (SPF) boosting properties in sun protection or make-up formulations.

Products based on renewable raw materials are of ever-increasing interest to consumers not just from an environmental perspective, but from a toxicological perspective too. For instance, partial esters of polyols with fatty acids have already made inroads into diverse fields of use in cosmetics and other areas. Corresponding products having "film-forming properties" are already on the market. Examples of these include:

Syncrowax™ ORM (Croda; INCI: Sorbitol/Sebacic Acid Copolymer Behenate),
Cera Bellina #106 (Koster Keunen; INCI: Polyglyceryl-3 Beeswax),
CosmoSurf® PG1-IS (Surfatech Corp.; INCI: Polyglyceryl-3 Stearate/Isostearate Dimer Dilinoleate Copolymer),
SurfaCare S (Surfatech Corp.; INCI: Oleic/Linoleic/Linolenic Polyglycerides),
LexFilm Sun natural (Inolex; INCI: Capryloyl Glycerin/Sebacic Acid Copolymer),
SolAmaze™ Natural polymer (Nouryon; INCI: Diisostearoyl Polyglyceryl-3 Dimer Dilinoleate (and) Caprylic/Capric Triglyceride),
Pelemol 6GPR (Phoenix Chem; INCI: Polyglyceryl-6 Polyricinoleate) and
Nomcort HK-G (INCI: Glyceryl Behenate/Eicosadioate), Nomcort HK-P (INCI: Polyglyceryl-10 Behenate/Eicosadioate) and Nomcort SG (INCI: Glyceryl Tribehenate/Isostearate/Eicosadioate) from Nisshin Oillio Group.

However, all these products have inadequate film-forming properties compared to the abovementioned synthetic/petrochemically based polymers.

Partial esters of polyglycerols are a particularly interesting group of products on account of the diverse possibilities for variation:
EP0835862 describes polyglycerol partial esters of saturated or unsaturated, linear or branched fatty acids and polyfunctional carboxylic acids, obtainable by esterification of a polyglycerol mixture with saturated or unsaturated, linear or branched fatty acids having 12 to 22 carbon atoms and polyfunctional carboxylic acids having 4 to 54 carbon atoms and an average functionality of 2 to 2.4, the degree of esterification of the polyglycerol mixture being between 30 and 75%, and the use thereof as W/O emulsifiers and dispersion aids.

A disadvantage of the specific polyglycerol partial esters disclosed in the prior art is that they are liquid at room temperature, albeit highly viscous, and are thus unsuitable as film formers at body temperature.

EP1500427 describes polyglycerol partial esters of polyhydroxystearic acid and polyfunctional carboxylic acids, obtainable by esterification of a polyglycerol mixture with polyhydroxystearic acid and dimer fatty acid and/or di- and/or tricarboxylic acids and fatty acids having 6 to 22 carbon atoms. The polyglycerol partial esters are used for low-viscosity W/O emulsions and as dispersion aids.

In a first step of the described preparation process, reactions of isostearic acid with polyglycerol and sebacic acid or a combination of sebacic acid and malic acid are described. The degree of esterification of the hydroxyl groups in this first step is here 19.9-63.4%.

A disadvantage of the method described in the prior art is that the products obtained are always highly viscous, i.e. but liquid at room temperature, even the product obtained at the end of the first step when the polyhydroxystearic acid is not added until the second step. Such products are unsuitable as film formers at body temperature.

EP1683781 describes polyglycerol partial esters of polyricinoleic acid and polyfunctional carboxylic acids, obtainable by esterification a) of a polyglycerol mixture with b) at least one particular polyricinoleic acid and optionally b1) polyhydroxystearic acid and c) at least one di- and/or tricarboxylic acid and d) at least one fatty acid according to methods known per se. The polyglycerol preferably has an average degree of condensation n of 1 to 11, preferably 2 to 6. In a first step of the described preparation process, reactions of isostearic acid with polyglycerol and sebacic acid are described. The degree of esterification of the hydroxyl groups in step 1 is here 36.5-49.0%. A disadvantage of the method described in the prior art is that the products obtained are always highly viscous, i.e. but liquid at room temperature, even the product obtained at the end of the first step when the polyhydroxystearic acid is not added until the second step. Such products are unsuitable as film formers at body temperature.

EP3500550 describes polyglycerol partial esters obtainable by esterification of a polyglycerol with a carboxylic acid mixture comprising at least one polyhydroxycarboxylic acid of a hydroxycarboxylic acid having 8 to 32 carbon atoms, at least one short-chain dicarboxylic acid having 2 to 16 carbon atoms, at least one long-chain dicarboxylic acid having 24 to 44 carbon atoms and at least one linear or branched, saturated or unsaturated fatty acid having 14 to 24 carbon atoms. These are used as W/O emulsifiers.

Reactions of monocarboxylic acid (oleic acid or isostearic acid) with polyglycerol and sebacic acid or dimer acid (examples 6a, 12 and 13) with a degree of esterification of 38.7-48.3% are described here. The products are liquids that are highly viscous at room temperature. Reactions of monocarboxylic acids (oleic acid, isostearic acid or palmitic/stearic acid) with polyglycerol and a combination of sebacic acid and dimer acid (examples 4, 6b and 14) with a degree of esterification of 48.7-61.6% are additionally described. With the exception of example 14, the products are highly viscous liquids at room temperature.

EP2593076 describes polyglycerol oligoesters obtainable by reacting polyglycerol having 3 to 20 glycerol units with a dicarboxylic acid or a cyclic anhydride of such a dicarboxylic acid having 4 to 22 carbon atoms and a monocarboxylic acid having 4 to 24 carbon atoms in a molar ratio of from 1.5:1.0:0.1 to 3.0:1.0:3.0. The products can be used as emulsifiers, solubilizing agents and/or thickeners.

The described examples have a degree of esterification of only 12.5-25.1%; according to the claims a degree of esterification of 66.6% is possible if using polyglycerol-3 in the ratio 1.5:1.0:3.0. The described examples have max. 2 equiv. of monocarboxylic acid (ex. E10) and always 2 equiv. of polyol (to 1 equiv. of dicarboxylic acid).

EP2363387 describes (poly)glycerol partial esters containing an average (numerical average) of 0.75 to 2.25 acid residues of one or more carboxylic acids having 10 to 24 carbon atoms and containing an average (numerical average) of 0.005 to 0.5 residues of a polyfunctional carboxylic acid. The use as O/W emulsifiers is described.

The reaction of polyglycerols having a degree of polymerization of 2.8-5.3 with fatty acids (C16, C18 and C22) and citric acid is described in the examples. The esters obtained have a degree of esterification of 27-43% of the hydroxyl groups of the polyglycerol used; no melting point data are given. The molar ratio of monocarboxylic acid to citric acid is 3.5-21.5.

EP1417955 describes a "humectant" consisting of (A) a glycerol/glycerol condensate ester with saturated linear fatty acids having 16-28 carbon atoms and a saturated aliphatic dicarboxylic acid having 16-28 carbon atoms, wherein at least half of the available hydroxyl groups of the glycerol/glycerol condensate remain free (unesterified), (B) a water-soluble diol and (C) a water-soluble tri- or polyol.

WO2015013951 describes a water-free cosmetic composition comprising (a) an ester of polyglycerol having an average degree of polymerization of 2-12, a linear saturated C16-28 fatty acid and an aliphatic saturated C16-28 dicarboxylic acid, (b) a polyol having at least 3 hydroxy groups and (c) a glyceryl ester surfactant.

All known products of the prior art exhibit only very minimal film-forming properties or none at all. They likewise exhibit little or no SPF-boosting properties. The water resistance, especially in sun protection formulations, is also inadequate.

Although synthetic polymers such as polyacrylates deliver significant effects, they are not based on natural raw materials and are also not biodegradable.

The skin feel of the known products of the prior art is unappealing, since corresponding cosmetic formulations that contain them are very tacky and/or are absorbed only slowly into the skin.

It was an object of the invention to provide a composition that is able to overcome at least one disadvantage of the products of the prior art.

<u>Description of the invention</u>

It has surprisingly been found that the polyglycerol partial esters described hereinbelow achieve the object of the invention.

The present invention accordingly provides polyglycerol partial esters that are characterized by a particular choice of polyglycerol and by specific ratios of the different carboxylic acids that have undergone esterification.

The invention further provides a method for preparing the polyglycerol partial esters of the invention and for the use of the polyglycerol partial esters in cosmetic applications.

An advantage of the present invention is that the polyglycerol partial esters described herein may be prepared from exclusively renewable raw materials, unlike the polyacrylates described above. A further advantage is that the polyglycerol partial esters described herein can be prepared on the basis of principles of green chemistry.

Another advantage of the present invention is that formulations can be provided that are polyglycol ether-free.

A further advantage is that the polyglycerol partial esters described herein are biodegradable, unlike the polyacrylates described above.

A further advantage is that the polyglycerol partial esters described herein have a good ecotoxicological profile.

A further advantage of the polyglycerol partial esters described herein is that they are very mild on the skin, not irritating and non-toxic.

Another advantage of the polyglycerol partial esters described herein is that they have improved sensory properties in formulations. The tackiness of sun protection formulations is reduced after application. The formulations undergo more rapid absorption into the skin, since absorption is increased during application and for 5 min thereafter on the skin.

Another advantage of the polyglycerol partial esters described herein is that they give rise to enhancement of "velvety-silkiness" in formulations.

A further advantage is that the polyglycerol partial esters described herein have a good skin-moisturizing effect.

Another advantage is that the polyglycerol partial esters described herein impart an increased sun protection factor to the sun protection formulations.

Another advantage is that the polyglycerol partial esters described herein are very well suited to be used in sun protection formulations having very high concentrations of UV light protection filters.

A further advantage of the polyglycerol partial esters described herein is that they impart very high water resistance to the formulations. In sun protection formulations this results in the formulations ensuring prolonged UV protection in the water or after bathing.

Another advantage of the polyglycerol partial esters according to the invention is that they impart increased wear resistance to the colour pigments when used in make-up applications.

In the context of pigment-containing formulations, a further advantage of the polyglycerol partial esters according to the invention is that they permit good dispersion of pigments in the formulations.

A further advantage of the polyglycerol partial esters described herein is that they have good compatibility with formulations containing UV protection filters or pigments.

In general, the polyglycerol partial esters described herein impart good stability to the formulations. Another advantage of the polyglycerol partial esters according to the invention is that they are easily processable, since they mix readily with typical cosmetic oils and can be rapidly incorporated into corresponding emulsions.

A further advantage of the polyglycerol partial esters described herein is that they impart high gloss to solid or waxy formulations such as lipsticks.

Yet another advantage of the polyglycerol partial esters according to the invention is that they are particularly tolerant to electrolytes, which means that, for example, formulations containing large amounts of salt remain stable.

A further advantage of the polyglycerol partial esters according to the invention is that they have a structuring and viscosity-increasing effect in formulations having high oil contents or even pure oils.

The present invention thus provides a polyglycerol partial ester obtainable by esterification

   a) of a polyglycerol having an average degree of condensation N of 2.4 to 15.0, preferably 2.6 to 10.0, especially 2.8 to 6.0,

with a carboxylic acid mixture comprising:

   b) at least one short-chain dicarboxylic acid having 4 to 18, preferably 4 to 14, more preferably 6 to 10, carbon atoms and

   c) at least one long-chain, saturated and linear monocarboxylic acid having 18 to 32, preferably 18 to 26, more preferably 18 to 22, carbon atoms,
   wherein the sum total of all short-chain dicarboxylic acids present in the carboxylic acid mixture comes to at least 75% by weight, preferably at least 85% by weight, more preferably at least 95% by weight, based on all the dicarboxylic acids present in the carboxylic acid mixture,
   wherein the sum total of all long-chain, saturated and linear monocarboxylic acids present in the carboxylic acid mixture comes to at least 86% by weight, preferably at least 90% by weight, more preferably at least 95% by weight, based on all the monocarboxylic acids present in the carboxylic acid mixture,
   wherein the molar ratio of all carboxyl groups present in the carboxylic acid mixture to all hydroxyl groups present in the polyglycerol used is at least 50 to 100, preferably at least 55 to 98, more preferably at least 68 to 95,
   characterized in that
   the molar ratio of polyglycerol a) to dicarboxylic acid component b) to carboxylic acid component c) is from 1.0:1.0:2.1 to 4.0:1.0:10.0, preferably from 1.0:1.0:3.1 to 3.0:1.0:8.0.

The polyglycerol partial esters according to the invention are mixtures of different substances; it is therefore clear to those skilled in the art that the numerical values specified are average values across the mixture.

The term "polyglycerol" is for the purpose of the present invention to be understood as meaning a polyglycerol that may also contain glycerol. Consequently, for the purposes of calculating amounts, masses and the like, any glycerol fraction should also be taken into consideration.

Because of its polymeric character, the polyglycerol is a statistical mixture of various compounds. Polyglycerol may have ether bonds formed between two primary, one primary and one secondary, and two secondary positions of the glycerol monomers. For this reason, the polyglycerol base framework does not usually consist exclusively of linearly linked glycerol units, but may also comprise branchings and rings. For details see for example "Original synthesis of linear, branched and cyclic oligoglycerol standards", Cassel et al., J. Org. Chem. 2001, 875-896.

The same applies to the term "polyglycerol partial ester" in the context of the present invention.

The term "short-chain dicarboxylic acid" is in the context of the present invention to be understood as meaning dicarboxylic acids having 4 to 18, preferably 4 to 14, more preferably 6 to 10, carbon atoms.

The term "long-chain" monocarboxylic acid is in the context of the present invention to be understood as meaning monocarboxylic acids having 18 to 32, preferably 18 to 26, more preferably 18 to 22, carbon atoms.

The polyglycerol partial ester obtainable by esterification of carboxylic acids according to the invention can of course also be obtained by esterification of the corresponding carboxylic acid derivatives, for example the anhydrides or carboxylic esters thereof (such as methyl or ethyl esters). If the polyglycerol partial esters are obtained by (trans) esterification of a polyol ester of the di- and/or monocarboxylic acid, it will be apparent to those skilled in the art that the molar ratio of polyglycerol a) to dicarboxylic acid component b) to carboxylic acid component c) relates not to the number of molecules of the respective polyol ester, but to the number of acyl residues of the di- and/or monocarboxylic acid provided by the respective polyol ester.

For the present invention, it is important that the polyglycerol used has an average degree of condensation $N$ of 2.4 to 15.0, preferably 2.6 to 10.0, especially 2.8 to 6.0.

The average degree of condensation of the polyglycerol $N$ is calculated via its hydroxyl value (OHV, in mg KOH/g) according to the following formula:

$$N = \frac{(112200 - 18 \cdot OHV)}{(74 \cdot OHV - 56100)}$$

Suitable detection methods for determining the hydroxyl value are in particular those according to DGF C-V 17 a (53), Ph. Eur. 2.5.3 Method A and DIN 53240.

Unless otherwise stated, all stated percentages (%) are percentages by weight.

Polyglycerol partial esters preferred in accordance with the invention are characterized in that the polyglycerol used has a glycerol content of 0.05% by weight to 25.0% by weight, preferably of 0.1% by weight to 15.0% by weight, more preferably of 0.1% by weight to 10.0% by weight, where the percentages by weight are based on the total amount of polyglycerol used. The stated percentages by weight are determined by the GC method described hereinbelow.

Polyglycerol partial esters preferred in accordance with the invention are characterized in that the polyglycerol used has a diglycerol content of 0.1% by weight to 45.0% by weight, preferably of 0.5% by weight to 35.0% by weight, more preferably of 3.0% by weight to 30.0% by weight, where the percentages by weight are based on the total amount of polyglycerol used.

The stated percentages by weight are determined by the GC method described hereinbelow.

It is advantageous when the polyglycerol used has a polydispersity index of more than 0.6, preferably of more than 1.0, more preferably of more than 1.2.

For the purposes of the present invention, the polydispersity index is calculated as

$$\sum_i |n_i - N| \cdot x_i$$

where $n_i$ is the degree of condensation of the individual oligomer i, N the average degree of condensation of the polyglycerol [already described above and how to determine] and $x_i$ the proportion of the oligomer i in the polyglycerol mixture, as determined in the GC method described hereinbelow.

A suitable method for determining the oligomer distribution of the polyglycerol in a given polyglycerol partial ester comprises the hydrolysis or alcoholysis of the partial ester, separation of the resulting polyglycerol from the carboxylic acid compounds formed and analysis by gas chromatography after derivatization.

For this purpose, 0.6 g of polyglycerol ester is boiled under reflux in 25 ml of 0.5 N ethanolic KOH for 30 minutes and adjusted to pH 2-3 with sulfuric acid. The carboxylic acids are separated by extracting with three portions of petroleum ether of equivalent volume. The combined extracts are evaporated to a volume of approx. 10 ml. A 0.5 ml aliquot is transferred to an autosampler vial and, after addition of 0.5 ml of MTBE and 1 ml of TMPAH solution (trimethylanilinium hydroxide in methanol) as derivatization agent, analysed by GC.

The fatty acid analysis by GC is carried out using a gas chromatograph equipped with a split/splitless injector, a capillary column and a flame ionization detector.

| Conditions: | | |
|---|---|---|
| Injector: | 290°C, split 30 ml | |
| Injected volume: | 1 $\mu$l | |
| Column: | 30 m * 0.32 mm HP1 0.25 $\mu$m | |
| Carrier gas: | Helium, inlet pressure 70 kPa | |
| Temp. program: | 80°C to 300°C at 8°C/min; | |
| Detector: | FID at | 320°C |
| | Hydrogen | 35 ml/min |
| | Air | 240 ml/min |
| | Make-up gas (purge gas) | 35 ml/min |

When using these conditions, the methyl carboxylate esters are separated according to their chain length.

The relative content of the individual carboxylic acids (chain-length distribution) is evaluated as a percentage of the peak area.

The residue after extraction with petroleum ether is adjusted to pH 7-8 by addition of barium hydroxide solution. The precipitated barium sulfate is removed by centrifugation. The supernatant is drawn off and the residue extracted with three 20 ml portions of ethanol. The combined supernatants are evaporated at 80°C/50 mbar. The residue is dissolved in pyridine. 500 $\mu$l of the solution is transferred to an autosampler vial and 1 ml of MSTFA (N-methyl-N-trifluoroacetamide) added. The vial is closed and heated to 80°C for 30 minutes.

The GC analysis of the polyglycerol component (as the trimethylsilyl derivative) is carried out using a gas-liquid chromatograph equipped with an on-column injector and an FID detector.

| Conditions: | |
|---|---|
| Injector: | On-column injection (direct injection onto column) |
| Injected volume: | 0.1 $\mu$l |
| Carrier gas: | 3 ml/min hydrogen (constant flow) |
| Column: | SimDist 12 m x 0.32 mm x 0.1 $\mu$m (Varian) |
| Temperature program: | 65°C to 365°C, 10°C/min |
| Detector (FID): | 375°C |

Under these conditions, the polyglycerols are separated according to their degree of condensation. In addition, cyclic isomers are separated from linear isomers up to a degree of condensation of four.

The peak areas of the individual oligomers are separated by a perpendicular line applied at the lowest point of the trough between peaks.

Since the resolution of oligomers higher than hexaglycerol is poor, the peaks of heptaglycerol and higher oligomers are combined as "heptaglycerol and higher" and treated as heptaglycerol for the purposes of calculating the polydispersity index. Linear and cyclic isomers are likewise combined in the calculation of the polydispersity index.

The relative ratio of the individual polyglycerol oligomers and isomers is calculated from the GC peak area obtained as described above.

The described GC analyses of the carboxylic acid and polyglycerol components can of course also be performed on the raw materials used for the preparation of the polyglycerol partial esters of the invention.

Polyglycerol partial esters preferred in accordance with the invention are characterized in that the polyglycerol used has a content of cyclic isomers of 1% by weight to 50% by weight, preferably of 2% by weight to 40% by weight, more preferably of 3% by weight to 30% by weight.

The stated percentages by weight are determined by the GC method described above and are based on the amount of the overall polyglycerol used.

It is preferable in accordance with the invention when the employed dicarboxylic acid in the polyglycerol partial ester of the invention is selected from aliphatic, linear dicarboxylic acids, especially succinic acid, maleic acid, tartaric acid, malic acid, fumaric acid, sorbic acid, α-ketoglutaric acid, glutaric acid, adipic acid, pimelic acid, suberic acid, azelaic acid, sebacic acid, undecanedioic acid, dodecanedioic acid and brassylic acid, with particular preference given to adipic acid, pimelic acid, suberic acid, azelaic acid and sebacic acid.

Polyglycerol partial esters preferred in accordance with the invention are characterized in that the long-chain, saturated and linear monocarboxylic acids used are selected from hydroxy-substituted and unsubstituted fatty acids.

When mixtures of hydroxy-substituted and unsubstituted fatty acids are in accordance with the invention used as long-chain, saturated and linear monocarboxylic acids, these fatty acid mixtures preferably have a content of hydroxy-substituted fatty acids within a range of from 0.1% by weight to 25% by weight, preferably from 1% by weight to 10% by weight, where the percentages by weight are based on all long-chain, saturated and linear monocarboxylic acids.

Polyglycerol partial esters particularly preferred in accordance with the invention are characterized in that the long-chain, saturated and linear monocarboxylic acids used are selected from unsubstituted fatty acids.

In this connection, the long-chain, saturated and linear monocarboxylic acids used are selected in particular from stearic acid, arachidic acid and behenic acid. Typical commercially available products are: technical-grade stearic acid containing at least 92% C18 (for example Mascid 1892 from Musim Mas, Palmac 90-18 from IOI, Radiacid 0152 from Oleon, Wilfarin SA-1892 from Wilmar), technical-grade stearic acid containing at least 98% C18 (for example Edenor C18-98 MY from Emery, Palmac 98-18 from IOI), technical-grade arachidic acid (for example Palmera A5020 from KLK, Nouracid RE07 from Oleon, Nouracid RD3030 from Oleon), technical-grade behenic acid containing at least 60% C22 (for example Palmera A6022 from KLK), technical-grade behenic acid containing at least 85% C22 (for example Palmera A8522 from KLK, Radiacid 0560 from Oleon) and technical-grade behenic acid containing at least 92% C22 (for example Palmera A9322 from KLK).

Polyglycerol partial esters preferred in accordance with the invention are characterized in that the total content of aromatic mono- and dicarboxylic acids based on all mono- and dicarboxylic acids used is from 0% by weight to 35% by weight, preferably from 0.1% by weight to 25% by weight. Polyglycerol partial esters particularly preferred in accordance with the invention are characterized in that the total content of aromatic mono- and dicarboxylic acids based on all mono- and dicarboxylic acids used is 0% by weight.

Polyglycerol partial esters alternatively particularly preferred are characterized in that the total content of aromatic mono- and dicarboxylic acids based on all mono- and dicarboxylic acids used is from 5% by weight to 35% by weight, preferably from 11% by weight to 25% by weight.

From the amounts of the various components used in the esterification, it is clearly evident that it is preferable in accordance with the invention when the polyglycerol partial ester of the invention has a degree of esterification of 50% to 100%, preferably of 55% to 98%, more preferably of 68% to 95%. The determination of the degree of esterification of the polyglycerol partial esters of the invention is described hereinbelow in the examples.

In this connection, it is particularly preferable that the polyglycerol partial ester of the invention has an acid value of less than 25, preferably less than 15, especially within a range from 0.1 to 10, more preferably within a range from 0.5 to 10.

For the present invention it is advantageous and thus preferable that the value for the hydrophilic-lipophilic balance (HLB value) of the polyglycerol partial ester is from 2.0 to 8.0, preferably from 2.5 to 6 and more preferably from 3 to 5.2. The HLB value is a measure of the degree of hydrophilicity or lipophilicity of the molecule determined through the calculation of values for the different regions of the molecule. For the purposes of the present invention, the HLB value of the polyglycerol partial esters is calculated as follows:

$$HLB = (mp/(mp+ma))*20,$$

where mp is the mass of the polyglycerol and ma the mass of the carboxylic acid mixture used in the synthesis of the polyglycerol ester (comprising mono- and dicarboxylic acid). For example, the esterification of 100 g of polyglycerol with 90 g of monocarboxylic acid and 10 g of dicarboxylic acid would result in an HLB value of (100 g / (90 g + 10 g + 100 g))*20 = 10, irrespective of the degree of polymerization of the polyglycerol and the nature of the carboxylic acids used.

Polyglycerol partial esters preferred in accordance with the invention are characterized in that they have an iodine value of less than 20, preferably less than 12, particularly preferably less than 5. A suitable method for determining the iodine value in the context of the present invention is EN 14111:2003.

It is preferable in accordance with the invention that the polyglycerol partial ester of the invention has a melting point of greater than 35°C, preferably greater than 40°C, in particular greater than 50°C, more preferably within a range from 51°C to 100°C.

The polyglycerol partial esters of the present invention can be prepared by classical esterification methods; in place of the carboxylic acids it is of course also possible to use the corresponding carboxylic acid derivatives, for example the anhydrides or carboxylic esters thereof (such as methyl or ethyl esters). It is also possible to use triglycerides, especially in the form of natural fats and oils, so long as they provide the carboxylic acids required according to the invention.

Polyglycerol partial esters according to the invention are preferably obtained by the method described hereinbelow; the present invention thus further provides a method for preparing a polyglycerol partial ester, said method comprising the method steps of

A) providing a polyglycerol having an average degree of condensation N of 2.4 to 15.0, preferably 2.6 to 10.0, especially 2.8 to 6.0,
B) providing a carboxylic acid mixture comprising:

at least one short-chain dicarboxylic acid having 4 to 18, preferably 4 to 14, more preferably 6 to 10, carbon atoms and
at least one long-chain, saturated and linear monocarboxylic acid having 18 to 32, preferably 18 to 26, more preferably 18 to 22, carbon atoms,
wherein the sum total of all short-chain dicarboxylic acids present in the carboxylic acid mixture comes to at least 75% by weight, preferably at least 85% by weight, more preferably at least 95% by weight, based on all the dicarboxylic acids present in the carboxylic acid mixture,
wherein the sum total of all long-chain, saturated and linear monocarboxylic acids present in the carboxylic acid mixture comes to at least 86% by weight, preferably at least 90% by weight, more preferably at least 95% by weight, based on all the monocarboxylic acids present in the carboxylic acid mixture,

C) esterifying the polyglycerol with the carboxylic acid mixture,

wherein the molar ratio of all carboxyl groups present in the carboxylic acid mixture to all hydroxyl groups present in the polyglycerol used is at least 50 to 100, preferably at least 55 to 98, more preferably at least 68 to 95, characterized in that the molar ratio of polyglycerol a) to dicarboxylic acid component b) to carboxylic acid component c) is from 1.0:1.0:2.1 to 4.0:1.0:10.0, preferably from 1.0:1.0:3.1 to 3.0:1.0:8.0.

In the method according to the invention, preference is given to using those polyglycerols, monocarboxylic acids and dicarboxylic acids that are cited above as used with preference for the polyglycerol partial ester according to the invention. Correspondingly graduated preferences can be applied by analogy.

In the method according to the invention, method step C) can be carried out as a one-pot reaction or else as a stepwise process.

If method step C) is carried out as a one-pot reaction, the carboxylic acid mixture is esterified with the polyglycerol.

If method step C) is carried out as a stepwise process, the monocarboxylic acid and the dicarboxylic acids are added sequentially and esterified with the polyglycerol; strictly speaking, the monocarboxylic acid and the dicarboxylic acid are not provided as a carboxylic acid mixture, but this process is equivalent. In this connection it is possible for the polyglycerol to first undergo reaction with the monocarboxylic acid before then undergoing crosslinking with the dicarboxylic acid in a second step. However, the reverse stepwise synthetic sequence comprising an initial reaction of the polyglycerol with the dicarboxylic acid and then a subsequent reaction with the monocarboxylic acid is possible too. Splitting the individual reactants into several portions and multistage reactions is another possible application.

Since the polyglycerol partial esters according to the invention have an excellent use profile in cosmetic formulations, the present invention further provides a formulation, especially a cosmetic formulation, comprising 1) the polyglycerol partial ester according to the invention or a polyglycerol partial ester obtainable by the method according to the invention.

Preferably, the formulation according to the invention comprises a further component 2) at least one substance selected from the group comprising UV light protection filter substances and pigments, especially UV light protection filter substances, which are preferably organic.

The UV light protection filter substances present may for example be organic substances that are capable of absorbing ultraviolet rays and re-emitting the absorbed energy in the form of longer-wavelength radiation, for example heat.

UVB filters can be oil-soluble or water-soluble. Examples of oil-soluble UVB light protection filters include: 3-benzylidenecamphor derivatives, for example 3-(4-methylbenzylidene)camphor (INCI: 4-Methylbenzylidene Camphor), 4-aminobenzoic acid derivatives, for example 2-ethylhexyl 4-(dimethylamino)benzoate (INCI: Ethylhexyl Dimethyl PABA), 2-hydroxyethyl 4-{bis[2-(2-hydroxyethoxy)ethyl]amino}benzoate (INCI: PEG-25 PABA), cinnamic esters, for example 2-ethylhexyl 4-methoxycinnamate (INCI: Ethylhexyl Methoxycinnamate), isopentyl 4-methoxycinnamate (INCI: Isoamyl p-Methoxycinnamate), 2-ethylhexyl 2-cyano-3,3-diphenylacrylate (INCI: Octocrylene), salicylic esters, for example 2-ethylhexyl salicylate (INCI: Ethylhexyl Salicylate), homomenthyl salicylate (INCI: Menthyl Salicylate), 3,3,5-trimethylcyclohexyl salicylate (INCI: Homosalate), 2-hydroxybenzoic acid - 2,2',2''-nitrilotriethanol (1:1) (INCI: TEA-Salicylate), benzophenone derivatives, for example 2-hydroxy-4-methoxybenzophenone (INCI: Benzophenone-3), 2,2'-dihydroxy-4-methoxybenzophenone (INCI: Benzophenone-8), 2-hydroxy-4-methoxy-4'-methylbenzophenone (INCI: Benzophenone-10), triazine derivatives, for example tris(2-ethylhexyl) 4,4',4''-(1,3,5-triazine-2,4,6-triyltriimino) tribenzoate (INCI: Ethylhexyl Triazone, obtainable for example under the trade name Uvinul T 150 from BASF), iscotrizinol (INCI: Diethylhexyl Butamido Triazone) and 2,4,6-tri(4-biphenylyl)-1,3,5-triazine (INCI: Tris-Biphenyl Triazine).

An additional UVB filter is the (3-(4-(2,2-bis(ethoxycarbonyl)vinyl)phenoxy)propenyl)methoxysiloxane/dimethylsiloxane copolymer (INCI: Polysilicone-15) obtainable for example under the trade name Parsol SLX.

Useful water-soluble UVB sun protection filters include for example:

salts of 2-phenylbenzimidazole-5-sulfonic acid, such as its alkali metal, alkaline earth metal, ammonium, alkylammonium, alkanolammonium and glucammonium salts, and also the sulfonic acid itself having the INCI name Phenylbenzimidazole Sulfonic Acid, sulfonic acid derivatives of benzophenone, for example 5-benzoyl-4-hydroxy-2-methoxybenzenesulfonic acid (INCI: Benzophenone-4) and salts thereof, benzenesulfonic acid derivatives of 3-benzylidenecamphor, for example 4-[(E)-(4,7,7-trimethyl-3-oxobicyclo[2.2.1]hept-2-ylidene)methyl]benzenesulfonic acid (INCI: Benzylidene Camphor Sulfonic Acid) and salts thereof.

Examples of typical oil-soluble UVA/broadband light protection filters that may be used include derivatives of benzoylmethane, for example 1-(4-methoxyphenyl)-3-[4-(2-methyl-2-propanyl)phenyl]-1,3-propanedione (INCI: Butyl Methoxydibenzoylmethane) or 1-(4-isopropylphenyl)-3-phenyl-1,3-propanedione (INCI: Isopropyl Dibenzoylmethane), triazine derivatives, for example 2,2'-[6-(4-methoxyphenyl)-1,3,5-triazine-2,4-diyl]bis{5-[(2-ethylhexyl)oxy]phenol} (INCI: Bis-Ethylhexyloxyphenol Methoxyphenyl Triazine, obtainable for example under the trade name Tinosorb S from BASF), N,N'-bis[4-[5-(1, 1-dimethylpropyl)-2-benzoxazolyl]phenyl]-N''-(2-ethylhexyl)-1,3,5-triazine-2,4,6-triamine (INCI: Ethylhexyl Bis-Isopentylbenzoxazolylphenyl Melamine, obtainable under the trade name Uvasorb K2A from 3V Sigma), and derivatives of benzophenone, for example hexyl 2-(4-diethylamino-2-hydroxybenzoyl)benzoate (INCI: Diethylamino Hydroxybenzoyl Hexyl Benzoate).

Useful water-soluble UVA/broadband light protection filters include, for example: 3,3'-(1,4-phenylenedimethylene)-bis(7,7-dimethyl-2-oxobicyclo[2.2.1]hept-1-ylmethanesulfonic acid) and salts thereof, especially the corresponding sodium, potassium or triethanolammonium salt, which is also described as benzene-1,4-di(2-oxo-3-bornylidenemethyl-10-sulfonic acid) and has the INCI name Terephthalylidene Dicamphor Sulfonic Acid (obtainable under the trade name Mexoryl SX), 2,2'-(1,4-phenylene)-bis(6-sulfo-1H-benzimidazole-4-sulfonic acid) and salts thereof, the corresponding sodium, potassium or triethanolammonium salts, for example disodium 2,2'-(1,4-phenylene)-bis(6-sulfo-1H-benzimidazole-4-sulfonate) having the INCI name Disodium Phenyl Dibenzimidazole Tetrasulfonate, trade name for example Neo Heliopan AP.

Examples of other UVA/broadband filters are 2,2'-methylenebis[6-(2H-benzotriazol-2-yl)-4-(2,4,4-trimethyl-2-pentanyl)phenol] (INCI: Methylene Bis-Benzotriazolyl Tetramethylbutylphenol, obtainable for example under the trade name Tinosorb M from BASF), 2-(2H-benzotriazol-2-yl)-4-methyl-6-[2-methyl-3-[1,3,3,3-tetramethyl-1-[(trimethylsilyl)oxy]disiloxanyl]propyl]phenol (INCI: Drometrizole Trisiloxane, trade name: Mexoryl XL), (1R,2S,5R)-2-isopropyl-5-methylcyclohexyl 2-aminobenzoate (INCI: Menthyl Anthranilate) and 2-ethoxyethyl (2E)-3-(4-methoxyphenyl)acrylate (INCI: Cinoxate).

The UV filters may of course also be present in mixtures in the compositions according to the invention.

In addition to the soluble UV light protection filter substances mentioned, insoluble pigments may also be used for this purpose, namely finely dispersed metal oxides or salts, for example titanium dioxide, zinc oxide, iron oxide, aluminium oxide, cerium oxide, zirconium oxide, silicates (talc), barium sulfate and zinc stearate. The particles should here have an average diameter of less than 100 nm, for example of between 5 and 50 nm and especially between 15 and 30 nm. They may be spherical in shape, but it is also possible to use particles that are ellipsoidal in shape or have a shape that deviates in another way from spherical. Also possible however are micronized organic pigments, for example 2,2'-methylenebis [6-(2H-benzotriazol-2-yl)-4-(2,4,4-trimethyl-2-pentanyl)phenol] (INCI: Methylene Bis-Benzotriazolyl Tetramethylbutylphenol, obtainable for example under the trade name Tinosorb M from BASF), having a particle size of < 200 nm, which is obtainable for example as a 50% aqueous dispersion.

In addition, further suitable UV light protection filters are given in the review by P. Finkel in SOFW Journal, 122, 543 (1996) or in the chapter "The Chemistry of Ultraviolet Filters" by N.A. Shaath in "Principles and Practice of Photoprotection", S.Q. Wang, H.W. Lim (eds.), Springer International Publishing, Switzerland, 2016.

It is preferable in accordance with the invention that component 2) of the formulation according to the invention is selected from at least two, preferably at least three, more preferably at least four, selected from the group of UV light protection filter substances.

It is preferable in accordance with the invention that component 2) of the formulation according to the invention is selected from the group comprising organic UV light protection filter substances, especially from the group of triazine derivatives.

It is in addition preferable in accordance with the invention that component 2) of the formulation according to the invention is selected from the group of UV light protection filter substances comprising, preferably consisting of,

> butyl methoxydibenzoylmethane,
> ethylhexyl triazone,
> bis-ethylhexyloxyphenol methoxyphenyl triazine,
> diethylhexyl butamido triazone,
> diethylamino hydroxybenzoyl hexyl benzoate,
> benzophenone-3,
> benzophenone-4,
> 4-methylbenzylidene camphor,
> octocrylene,
> ethylhexyl methoxycinnamate,
> ethylhexyl salicylate,
> homomenthyl salicylate,
> phenylbenzimidazole sulfonic acid,
> methylene bis-benzotriazolyl tetramethylbutylphenol,
> disodium phenyl dibenzimidazole tetrasulfonate,
> isoamyl p-methoxycinnamate,
> ethylhexyl dimethyl PABA.

It is in addition preferable in accordance with the invention that component 2) of the formulation according to the invention is selected from the group of pigments comprising unmodified or surface-modified, inorganic or organic pigments, preferably inorganic pigments such as activated carbon, talc, iron oxide pigments, titanium dioxide, zinc oxide, silica, cerium oxides, zirconium oxides, aluminium oxides, calcium carbonate, barium sulfate, chromium oxides, manganese oxides, bismuth oxychloride, ultramarine, calcium sulfate, alkali magnesium silicates or mixtures thereof.

Examples of organic pigments may be: carotenoids, chlorophylls, xanthophylls, caramel and the salts of the recited examples and also dyes obtained from fruits of plants or other plant parts, for example from orange, cacao, turmeric, shea, sandalwood, onion, carob tree fruit, paprika, maize, tomato, beetroot, peanut, grape, red cabbage, red rice, radish, elderberry, lingonberry, blueberry, raspberry, blackberry, boysenberry, gooseberry, cranberry, saffron, strawberry, cherry, tea, hibiscus, plum, blueberry or mulberry. Also employable as dye pigments are the substances approved and suitable for cosmetic purposes, as listed for example in the publication "Kosmetische Färbemittel" [Cosmetic colorants] of the Dyes Committee of the Deutsche Forschungsgemeinschaft [German Research Foundation], Verlag Chemie, Weinheim, 1984, pages 81 to 106.

It is in addition preferable in accordance with the invention that, when component 2) of the formulation according to the invention is selected from the group of UV light protection filter substances, pigments selected from titanium dioxide and zinc oxide are additionally present as component 2. In this connection too, the UV light protection filter substances that are preferably present correspond to the preferred UV light protection filter substances mentioned above.

A preferred formulation according to the invention is characterized in that

> component 1) is present in an amount of 0.1% by weight to 20% by weight, preferably of 0.25% by weight to 12% by weight, more preferably of 0.5% by weight to 6% by weight, and
> component 2) is present in an amount of 0.1% by weight to 60% by weight, preferably of 1% by weight to 50% by weight, more preferably of 3% by weight to 40% by weight.

The formulations according to the invention can comprise for example at least one further additional component selected from the group comprising

> emollients,
> emulsifiers,
> co-emulsifiers,
> thickeners/viscosity regulators/stabilizers,
> antioxidants,
> hydrotropes (or polyols),
> solids and fillers,
> pearlescent additives and opacifiers,
> insect repellents,
> self-tanning agents,
> preservatives,

conditioning agents,
perfumes,
colorants,
cosmetic active substances,
care additives,
refatting agents,
solvents.

Substances that can be used as exemplary representatives of the individual groups are known to those skilled in the art and can for example be taken from German application DE102008001788.4.

As regards further optional components and also the amounts used of these components, reference is expressly made to the relevant handbooks known to those skilled in the art, for example K. Schrader, "Grundlagen und Rezepturen der Kosmetika" [Fundamentals and formulations of cosmetics], 2nd edition, pages 329 to 341, Hüthig Buch Verlag, Heidelberg. The amounts of the respective additives depend on the intended use.

Typical starting formulations for the relevant applications are known prior art and are contained for example in the brochures of the manufacturers of the relevant base materials and active substances. These existing formulations can generally be adopted unchanged. However, any desired modifications necessary for adjustment and optimization can be made in a straightforward manner through simple tests.

Formulations according to the invention may for example be used in the form of an emulsion, a suspension, a solution, a cream, a salve, a paste, a gel, an oil, a powder, an aerosol, a stick, a spray, a cleansing product, a make-up product or a sun protection product.

The present invention further provides for the use of the polyglycerol partial esters of the invention, or of the polyglycerol partial esters obtainable by the method of the invention, as film formers.

The present invention further provides for the use of the polyglycerol partial esters of the invention, or of the polyglycerol partial esters obtainable by the method of the invention, to boost the sun protection factor of UV light protection filter substances.

The present invention further provides for the use of the polyglycerol partial esters of the invention, or of the polyglycerol partial esters obtainable by the method of the invention, to reduce the rinseability and/or the wear of a formulation from a surface.

In the use according to the invention, preference is given to using the components mentioned above as components that are preferably present in the context of the formulations according to the invention.

The examples that follow describe the present invention by way of example without any intention to limit the invention, the scope of application of which is apparent from the entirety of the description and the claims, to the embodiments specified in the examples.

Examples:

*Melting point determination by the capillary method:*

Melting points were determined using the capillary method based on DIN 53181, DIN EN ISO 3146, DGF C-IV 3a and Ph. Eur. 2.2.14.

*Degree of esterification:*

The degree of esterification of all OH groups can be determined via the hydroxyl value, the acid value and the saponification value according to the following formula:

$$\text{Degree of esterification} = 100\,(SV - AV)\,/\,(SV - AV + OHV)$$

where SV = saponification value, AV = acid value and OHV = hydroxyl value.

Suitable detection methods for determining the saponification value are in particular those according to DGF C-V 3, DIN EN ISO 3681 and Ph. Eur. 2.5.6.

Suitable methods for determining the acid value are in particular those according to DGF C-V 2, DIN EN ISO 2114, Ph. Eur. 2.5.1, ISO 3682 and ASTM D 974.

Suitable detection methods for determining the hydroxyl value are in particular those according to DGF C-V 17 a (53), Ph. Eur. 2.5.3 Method A and DIN 53240.

*Ratio of the raw materials:*

The molar ratio of polyglycerol a) to dicarboxylic acid component b) to carboxylic acid component c) is abbreviated to PG:Di:Mono.

*Example 1, inventive: Polyglycerol partial ester (PGE), inventive*

A mixture of polyglycerol (OHV = 1100 mg KOH/g, 50.0 g, 0.208 mol), stearic acid (92%, 284 g/mol, AV = 199 mg KOH/g, 155.4 g, 0.547 mol) and sebacic acid (202 g/mol, 35.3 g, 0.174 mol) was heated to 240°C with stirring and the water that formed was continuously distilled off until an acid value of approx. 5 mg KOH/g was attained. After cooling, a light yellow, brittle, solid product was obtained.
Acid value: 4.4 mg KOH/g; saponification value: 229 mg KOH/g; hydroxyl value: 29 mg KOH/g; degree of esterification: 88.6%; melting point: 52°C; HLB: 4.2.
PG:Di:Mono = 1.2:1:3.1

*Example 2, inventive: PGE, inventive*

A mixture of polyglycerol (OHV = 1100 mg KOH/g, 50.0 g, 0.208 mol), stearic acid (92%, 284 g/mol, AV = 199 mg KPH/g, 197.8 g, 0.696 mol) and sebacic acid (202 g/mol, 20.2 g, 0.100 mol) was heated to 240°C with stirring and the water that formed was continuously distilled off until an acid value of approx. 5 mg KOH/g was attained. After cooling, a light yellow, brittle, solid product was obtained.
Acid value: 5.1 mg KOH/g; saponification value: 215 mg KOH/g; hydroxyl value: 30 mg KOH/g; degree of esterification: 87.5%; melting point: 55°C; HLB: 3.7.
PG:Di:Mono = 2.1:1:7.0.

*Example 3, inventive: PGE, inventive*

A mixture of polyglycerol (OHV = 1100 mg KOH/g, 50.0 g, 0.208 mol), behenic acid (92%, 340 g/mol, 187.8 g, 0.55 mol) and sebacic acid (202 g/mol, 35.3 g, 0.175 mol) was heated to 240°C with stirring and the water that formed was continuously distilled off until an acid value of approx. 5 mg KOH/g was attained. After cooling, a light yellow, brittle, solid product was obtained.
Acid value: 3.3 mg KOH/g; saponification value: 197 mg KOH/g; hydroxyl value: 32 mg KOH/g; degree of esterification: 85.4%; melting point: 66°C; HLB: 3.7.
PG:Di:Mono = 1.2:1:3.1.

*Example 4, inventive: PGE, inventive*

A mixture of polyglycerol (OHV = 1100 mg KOH/g, 50.0 g, 0.208 mol) and stearic acid (92%, 284 g/mol, AV = 199 mg KOH/g, 197.8 g, 0.696 mol) was heated to 240°C with stirring and the water that formed was continuously distilled off until an acid value of less than 20 mg KOH/g was attained. After addition of succinic acid (118 g/mol, 11.7 g, 0.099 mol), the mixture was further heated to 240°C with stirring and the water that formed was continuously distilled off until an acid value of less than 5 mg KOH/g was attained. After cooling, a light yellow, brittle, solid product was obtained.
Acid value: 2.9 mg KOH/g; saponification value: 207 mg KOH/g; hydroxyl value: 39 mg KOH/g; degree of esterification: 84.0%; melting point: 58°C; HLB: 3.9.
PG:Di:Mono = 2.1:1:7.0.

*Example 5, inventive: PGE, inventive*

A mixture of polyglycerol (OHV = 1100 mg KOH/g, 50.0 g, 0.208 mol) and stearic acid (92%, 284 g/mol, AV = 199 mg KOH/g, 155.4 g, 0.547 mol) was heated to 240°C with stirring and the water that formed was continuously distilled off until an acid value of less than 20 mg KOH/g was attained. After addition of adipic acid (146 g/mol, 25.5 g, 0.175 mol), the mixture was further heated to 240°C with stirring and the water that formed was continuously distilled off until an acid value of less than 5 mg KOH/g was attained. After cooling, a light yellow-brown, brittle, solid product was obtained.
Acid value: 2.0 mg KOH/g; saponification value: 241 mg KOH/g; hydroxyl value: 31 mg KOH/g; degree of esterification: 88.5%; melting point: 53°C; HLB: 4.3.
PG:Di:Mono = 1.2:1:3.1.

*Example 6, inventive: PGE, inventive*

A mixture of polyglycerol (OHV = 1100 mg KOH/g, 50.0 g, 0.208 mol), stearic acid (92%, 284 g/mol, AV = 199 mg KOH/g, 155.4 g, 0.547 mol) and azelaic acid (188 g/mol, 34.1 g, 0.181 mol) was heated to 240°C with stirring and the water that formed was continuously distilled off until an acid value of approx. 5 mg KOH/g was attained. After cooling, a light yellow, brittle, solid product was obtained.

Acid value: 2.7 mg KOH/g; saponification value: 239 mg KOH/g; hydroxyl value: 35 mg KOH/g; degree of esterification: 87.1%; melting point: 54°C; HLB: 4.2.

PG:Di:Mono = 1.2:1:3.1.

*Example 7, inventive: PGE, inventive*

A mixture of polyglycerol (OHV = 1100 mg KOH/g, 50.0 g, 0.208 mol), stearic acid (92%, 284 g/mol, AV = 199 mg KOH/g, 169.6 g, 0.597 mol) and brassylic acid (244 g/mol, 36.5 g, 0.149 mol) was heated to 240°C with stirring and the water that formed was continuously distilled off until an acid value of approx. 5 mg KOH/g was attained. After cooling, a light yellow, brittle, solid product was obtained.

Acid value: 3.7 mg KOH/g; saponification value: 217 mg KOH/g; hydroxyl value: 30 mg KOH/g; degree of esterification: 87.7%; melting point: 54°C; HLB: 3.9.

PG:Di:Mono = 1.4:1:4.0.

*Example 8, inventive: PGE, inventive*

A mixture of polyglycerol (OHV = 1100 mg KOH/g, 50.0 g, 0.208 mol) and stearic acid (92%, 284 g/mol, AV = 199 mg KOH/g, 169.6 g, 0.597 mol) was heated to 240°C with stirring and the water that formed was continuously distilled off until an acid value of less than 20 mg KOH/g was attained. After addition of tetradecanedicarboxylic acid (258 g/mol, 38.6 g, 0.149 mol), the mixture was further heated to 240°C with stirring and the water that formed was continuously distilled off until an acid value of less than 5 mg KOH/g was attained. After cooling, a light yellow, brittle, solid product was obtained.

Acid value: 2.0 mg KOH/g; saponification value: 206 mg KOH/g; hydroxyl value: 27 mg KOH/g; degree of esterification: 88.3%; melting point: 53°C; HLB: 3.9.

PG:Di:Mono = 1.4:1:4.0.

*Example 9, inventive: PGE, inventive*

A mixture of polyglycerol (OHV = 1100 mg KOH/g, 64.7 g, 0.270 mol) and stearic acid (92%, 284 g/mol, AV = 199 mg KOH/g, 146.2 g, 0.515 mol) was heated to 240°C with stirring and the water that formed was continuously distilled off until an acid value of less than 20 mg KOH/g was attained. After addition of sebacic acid (202 g/mol, 39.1 g, 0.193 mol), the mixture was further heated to 240°C with stirring and the water that formed was continuously distilled off until an acid value of less than 5 mg KOH/g was attained. After cooling, a light yellow, brittle, solid product was obtained.

Acid value: 1.2 mg KOH/g; saponification value: 221 mg KOH/g; hydroxyl value: 101 mg KOH/g; degree of esterification: 68.5%; melting point: 51°C; HLB: 5.2.

PG:Di:Mono = 1.4:1:2.7.

*Example 10, inventive: PGE, inventive*

A mixture of a first polyglycerol ("polyglycerol-3", OHV = 1100 mg KOH/g, 33.7 g, 0.140 mol) and a second polyglycerol ("polyglycerol-6", OHV = 972 mg KOH/g, 31.6 g, 0.070 mol), behenic acid (92%, 340 g/mol, 238.0 g, 0.700 mol) and sebacic acid (202 g/mol, 20.2 g, 0.100 mol) was heated to 240°C with stirring and the water that formed was continuously distilled off until an acid value of approx. 5 mg KOH/g was attained. After cooling, a light yellow, brittle, solid product was obtained. Acid value: 5.0 mg KOH/g; saponification value: 191 mg KOH/g; hydroxyl value: 33 mg KOH/g; degree of esterification: 84.9%; melting point: 61°C; HLB: 4.0.

PG:Di:Mono = 2.1:1:7.0.

*Example 11, noninventive: PGE, palmitate*

A mixture of polyglycerol (OHV = 1100 mg KOH/g, 53.5 g, 0.223 mol), palmitic acid (256 g/mol, 164.1 g, 0.640 mol) and sebacic acid (202 g/mol, 32.3 g, 0.160 mol) was heated to 240°C with stirring and the water that formed was continuously distilled off until an acid value of < 5 mg KOH/g was attained. After cooling, a light yellow, brittle, solid product was obtained.

Acid value: 1.9 mg KOH/g; saponification value: 236 mg KOH/g; hydroxyl value: 36 mg KOH/g; degree of esterification: 86.7%; melting point: 44°C; HLB: 4.3.
PG:Di:Mono = 1.4:1:4.0.

*Example 12, noninventive: PGE without dicarboxylic acid*

A mixture of polyglycerol (OHV = 1100 mg KOH/g, 50.0 g, 0.208 mol) and stearic acid (92%, 284 g/mol, AV = 199 mg KOH/g, 254.3 g, 0.895 mol) was heated to 240°C with stirring and the water that formed was continuously distilled off until an acid value of < 5 mg KOH/g was attained. After cooling, a yellowish, brittle, solid product was obtained.
Acid value: 3.8 mg KOH/g; saponification value: 176 mg KOH/g; hydroxyl value: 24 mg KOH/g; degree of esterification: 87.8%; melting point: 58°C; HLB: 3.3.

*Example 13, noninventive: Reworking of example E10 from EP2593076, low degree of esterification*

A mixture of polyglycerol (462 g/mol, 160.0 g, 0.346 mol) and succinic acid (118 g/mol, 20.4 g, 0.173 mol) with addition of catalyst KOH (0.67 g) was heated to 240°C with stirring and the water that formed was continuously distilled off until an acid value of < 5 mg KOH/g was attained. After addition of palmitic acid (256 g/mol, 88.7 g, 0.346 mol), the mixture was further heated to 240°C with stirring and the water that formed was continuously distilled off until an acid value of less than 5 mg KOH/g was attained. After cooling, a yellowish, soft, solid product was obtained.
Acid value: 3.1 mg KOH/g; saponification value: 149 mg KOH/g; hydroxyl value: 658 mg KOH/g; degree of esterification: 18.2%; HLB: 11.9.
PG:Di:Mono = 2.0:1:2.0.

*Example 14, noninventive: similar to example E14 from EP2593076, low degree of esterification*

A mixture of polyglycerol (759 g/mol, 258.1 g, 0.277 mol), succinic acid (118 g/mol, 16.3 g, 0.138 mol) and stearic acid (92%, 284 g/mol, 29.5 g, 0.104 mol) with addition of catalyst hypophosphoric acid (0.64 g) was heated to 240°C with stirring and the water that formed was continuously distilled off until an acid value of < 5 mg KOH/g was attained. After cooling, a yellowish, solid product was obtained.
Acid value: 0.7 mg KOH/g; saponification value: 88 mg KOH/g; hydroxyl value: 482 mg KOH/g; degree of esterification: 15.3%; HLB: 17.0.
PG:Di:Mono = 2.0:1:0.75.

*Example 15, noninventive: Reworking of example 6A (first process step) from EP3500550, isostearic acid*

A mixture of polyglycerol (315 g/mol, 120.1 g, 0.381 mol), sebacic acid (202 g/mol, 31.5 g, 0.156 mol) and isostearic acid (285 g/mol, 164.4 g, 0.577 mol) was heated to 240°C with stirring and the water that formed was continuously distilled off until an acid value of < 5 mg KOH/g was attained. After cooling, a clear, light yellow, liquid product was obtained.
Acid value: 0.3 mg KOH/g; saponification value: 167 mg KOH/g; hydroxyl value: 261 mg KOH/g; degree of esterification: 39.0%; HLB: 7.6.
PG:Di:Mono = 2.4:1:3.7.

*Example 16 a-g, further noninventive commercial products*

   a. Tego SP 13 Sun Up MB (Evonik, INCI: Poly C10-30 Alkyl Acrylate)
   b. Antaron V-220 (Ashland, INCI: VP/Eicosene Copolymer)
   c. Isolan PDI (Evonik, INCI: Diisostearoyl Polyglyceryl-3 Dimer Dilinoleate)
   \d. Isolan GPS (Evonik, INCI: Polyglyceryl-4 Diisostearate/Polyhydroxystearate/Sebacate)
   e. Cera Bellina #106 (Koster Keunen; INCI: Polyglyceryl-3 Beeswax)
   f. CosmoSurf PG1-IS (Surfatech Corp.; INCI: Polyglyceryl-3 Stearate/Isostearate Dimer Dilinoleate Copolymer)
   g. LexFilm™ Sun Natural MB (Inolex, INCI: Capryloyl Glycerin/Sebacic Acid Copolymer)

*Example 17, inventive: PGE, inventive*

A mixture of polyglycerol (OHV = 1125 mg KOH/g, 90.0 g, 0.335 mol) and stearic acid (92%, 284 g/mol, AV = 199 mg KOH/g, 210 g, 0.738 mol) was heated to 240°C with stirring and the water that formed was continuously distilled off until an acid value of less than 2 mg KOH/g was attained. After addition of sebacic acid (202 g/mol, 42.1 g, 0.208 mol) at 130°C, the mixture was further reacted with stirring at 240°C under reduced pressure and the water continuously distilled off until an acid value of <50 mg KOH/g was attained. After cooling, a light yellow, solid product was obtained.

Acid value: 31 mg KOH/g; saponification value: 215 mg KOH/g; hydroxyl value: 130 mg KOH/g; degree of esterification: 58.6%; melting point: 51 °C; HLB: 5.3.

PG:Di:Mono = 1.6:1:3.5

*Example 18, noninventive: example 1 from* EP2363387*, citric acid*

This product example with citric acid was obtained according to the procedure described in example 1 of EP2363387.

*Application example 1: In-vitro SPF-boosting test of cosmetic formulations*

Polyglycerol partial esters (PGEs) according to the invention have the characteristic feature of a strong tendency to film formation compared to noninventive polyglycerol partial esters or noninventive commercial products. The film-forming properties in sun protection formulations can be quantitatively demonstrated by means of in-vitro SPF tests.

This was done by preparing O/W sun protection emulsions on a 200 g laboratory scale into which were incorporated the various film formers in an each time identical manner according to the recipes hereinbelow (Table 1). The emulsions were prepared according to the method known to those skilled in the art commonly used in the production of lotions.

Table 1: Composition of the O/W sun protection emulsions for in-vitro determination of the SPF

| Phase | Ingredient | Formulation A (reference) | Formulation B |
|---|---|---|---|
| A | Tego® Care PBS 6 MB (Polyglyceryl-6 Stearate, Polyglyceryl-6 Behenate) | 3.00% | 3.00% |
| | Tegin® M Pellets MB (Glyceryl Stearate) | 0.50% | 0.50% |
| | Tego® Alkanol 1618 (Cetearyl Alcohol) | 0.50% | 0.50% |
| | Tegosoft® XC MB (Phenoxyethyl Caprylate) | 5.00% | 3.50% |
| | Bis-Ethylhexyloxyphenol Methoxyphenyl Triazine | 3.00% | 3.00% |
| | Butyl Methoxydibenzoylmethane | 2.00% | 2.00% |
| | Ethylhexyl Salicylate | 3.00% | 3.00% |
| | Octocrylene | 8.00% | 8.00% |
| | Homosalate | 3.00% | 3.00% |
| | Ethylhexyl Triazone | 1.50% | 1.50% |
| | dermofeel® Toco 70 non GMO (Tocopherol, Helianthus Annuus (Sunflower) Seed Oil) | 0.70% | 0.70% |
| | **PGE inventive/noninventive/commercial product** | - | **1.50%** |
| B | Glycerin | 3.00% | 3.00% |
| | Disodium EDTA | 0.05% | 0.05% |
| | Water | to 100% | to 100% |
| C | Xanthan Gum (Keltrol CG-SFT, CP Kelco) | 0.50% | 0.50% |
| D | Verstatil® PC (Phenoxyethanol, Caprylyl Glycol) | 1.20% | 1.20% |

24.5 mg of reference formulation A and of test formulations B were respectively evenly applied to and spread over polymethyl methacrylate plates (PMMA; 7.0 cm x 3.5 cm, 2 $\mu$m roughness, Schönberg GmbH & Co. KG) (1 mg/cm$^2$). For each measurement, six replicate determinations were carried out, i.e. each formulation was applied to 6 individual PMMA plates. These were left in a drying cabinet at 30°C for 30 min, after which the SPF of the plates was determined using a Labsphere UV2000S Ultraviolet Transmittance Analyzer in accordance with Colipa Guideline (2011) with in each case 4 measurement points per PMMA plate. The individual SPF values for each PMMA plate/formulation were noted and the mean value determined. The mean SPF values for each formulation with film former were divided by the mean SPF value of formulation A without film former. The results are listed below in Table 2.

Table 2: Relative in-vitro SPF values of the example products in formulation B

| Formulation B | Product examples present in formulation B | SPF boost (relative to reference formulation A) |
|---|---|---|
| B1 | Tego® SP 13 Sun Up (Poly C10-30 Alkyl Acrylate) | 1.94 |
| B2 | Antaron V-220 (Ashland, INCI: VP/Eicosene Copolymer) | 1.55 |
| B3 | Isolan® PDI (Diisostearoyl Polyglyceryl-3 Dimer Dilinoleate) | 1.15 |
| B4 | Isolan® GPS (Polyglyceryl-4 Diisostearate/ Polyhydroxystearate/Sebacate) | 1.16 |
| B5 | Polyglyceryl-3 Beeswax (Cera Bellina, Koster Keunen) | 1.39 |
| B6 | Polyglyceryl-3 Stearate/Isostearate/Dimer Dilinoleate Crosspolymer (CosmoSurf PG1-IS, SurfaTech Corp.) | 1.01 |
| B7 | Capryloyl Glycerin/Sebacic Acid Copolymer (LexFilm Sun Natural MB, Inolex Inc.) | 1.00 |
| B8 | Ex. 11 | 1.20 |
| B9 | Ex. 12 | 1.06 |
| B10 | Ex. 13 (= example E10 from EP2593076) | 1.02 |
| B11 | Ex. 14 (= example E14 from EP2593076) | 1.02 |
| B12 | Ex. 15 (= example 6A (first process step only) from EP3500550) | 1.00 |
| B13* | Ex. 1 | 1.94 |
| B14* | Ex. 2 | 1.64 |
| B15* | Ex. 3 | 1.81 |
| B16* | Ex. 4 | 1.44 |
| B17* | Ex. 5 | 1.89 |
| B18* | Ex. 6 | 1.90 |
| B19* | Ex. 7 | 1.74 |
| B20* | Ex. 8 | 1.94 |
| B21* | Ex. 9 | 1.60 |
| B22* | Ex. 10 | 1.61 |
| B23* | Ex. 17 | 1.9 |
| B24 | Ex. 18 (= example 1 from EP2363387) | 1.4 |
| * inventive examples | | |

For the inventive examples, the results in Table 2 in all cases show SPF-boost values of over 1.4 (relative to reference formulation A without film former). Indeed, values comparable with the polyacrylate Tego® SP 13 Sun Up and better than with the synthetic VP/Eicosene Copolymer were achieved by the best polyglycerol partial esters according to the invention. Significantly better values compared to plant-based reference substances of the prior art were obtained.

*Application example 2: In-vivo SPF water resistance test of cosmetic formulations*

To determine the water resistance of sun protection formulations and sun protection factors (SPF) thereof, screening studies in accordance with Colipa 2005 (Cosmetics Europe: Guidelines For Evaluating Sun Product Water Resistance, 2005) were carried out at an external, accredited test institute.

The sun protection factor of a test person (SPFi) before and after water-bath treatment is calculated as the ratio of the minimum erythema dose on protected skin (MEDp) and the minimum erythema dose on unprotected skin (MEDu) on the same test person: SPFi = MEDp/MEDu. The minimum erythema dose is defined here as the amount of energy necessary to produce the first clearly perceptible redness within a clearly defined area, the redness being assessed 16 h to 24 h after exposure to light using a sunlight simulator (6 increasing UV doses with 15% progression). The sun protection factor is determined after application of 2 $\pm$ 0.05 mg of the test formulation on 1 cm$^2$ of skin before and after two periods of immersion in a water bath at 30 $\pm$ 2°C for 20 min each time. The water resistance is then obtained as the ratio of the sun protection factor after and before the water bath: WR = SPF(after water bath)/SPF(before water bath). For further retails, reference is made to the method description of the Colipa (The European Cosmetic and Perfumery Association).

Table 3: Composition of the O/W sun protection emulsions for in-vivo measurement of the SPF water resistance

| Phase | Ingredient | Formulation C (reference) % w/w | Formulation D % w/w | Formulation E % w/w |
|---|---|---|---|---|
| A | Tego® Care PBS 6 MB (Polyglyceryl-6 Stearate, Polyglyceryl-6 Behenate) | 3.00 | 3.00 | 3.00 |
| | Tegin® M Pellets MB (Glyceryl Stearate) | 0.50 | 0.50 | 0.50 |
| | Tego® Alkanol 1618 (Cetearyl Alcohol) | 0.50 | 0.50 | 0.50 |
| | Tegosoft® XC MB (Phenoxyethyl Caprylate) | 6.00 | 4.50 | 4.50 |
| | Antaron V-220 (Ashland, INCI: VP/Eicosene Copolymer) | - | 1.50 | - |
| | **Polyglycerol partial ester (PGE) Ex. 1** | - | - | **1.50** |
| | Bis-Ethylhexyloxyphenol Methoxyphenyl Triazine (Tinosorb S, BASF) | 3.00 | 3.00 | 3.00 |
| | Butyl Methoxydibenzoylmethane | 2.00 | 2.00 | 2.00 |
| | Ethylhexyl Salicylate | 3.00 | 3.00 | 3.00 |
| | Octocrylene | 8.00 | 8.00 | 8.00 |
| | Homosalate | 3.00 | 3.00 | 3.00 |
| | Ethylhexyl Triazone | 1.50 | 1.50 | 1.50 |
| B | Water | to 100 | to 100 | to 100 |
| | Glycerin | 3.00 | 3.00 | 3.00 |
| C | Acrylates/C10-30 Alkyl Acrylate Crosspolymer (Carbomer Copolymer C) | 0.20 | 0.20 | 0.20 |
| | Tegosoft® XC MB (Phenoxyethyl Caprylate) | 0.80 | 0.80 | 0.80 |
| D | Sodium hydroxide (10% in water) | 0.60 | 0.60 | 0.60 |
| E | Verstatil® PC (Phenoxyethanol, Caprylyl Glycol) | 1.00 | 1.00 | 1.00 |

Table 4: In-vivo SPF water resistance values for formulation C, D and E

| Formulation | C | D | | E |
|---|---|---|---|---|
| Film-former product example in formulation | - | Antaron V-220 (Ashland, INCI: VP/Eicosene Copolymer) | | PGE Ex. 1 |
| In-vivo water resistance (WR, in %) | 34.6 | 58.8 | | 58.4 |

Table 4 details the results of the in-vivo water resistance measurements with formulations C, D and E from Table 3. The vehicle formulation C (without film former) showed a WR value of only 34.6% and cannot therefore be considered a water-resistant sun protection formulation in cosmetics. By contrast, formulation E with inventive example 1 provided good water resistance, with a WR value of 58.4%, and could be declared "water resistant" (i.e. WR of min. 50%). The addition of the inventive product example 1 thus brought about a significant and very good improvement in the water resistance of formulation C and is at the same level as the comparative formulation D with the synthetic comparative example VP/Eicosene Copolymer.

*Application example 3: Sensory properties test of cosmetic formulations*

The test formulations V1, V2, V3, V4, V5, V6 and V7 were investigated by a group of volunteer subjects trained in the assessment of sensory properties (N = 13). The skin feel of the cosmetic formulations described in the examples in Tables 5a and 5b was determined by a so-called panel. In these tests, a defined amount of the test lotions was applied to a clearly defined area of the forearm. The volunteer subjects compared the sensory properties of the cosmetic formulations and of the respective comparative formulation without knowing the composition. Assessment was on a scale from 0 (little) to 10 (much).

Inventive examples are marked with an *.

Table 5a: Composition of the sun protection formulations for the sensory properties test

| | | | V1 | V2* | V3 |
|---|---|---|---|---|---|
| **Phase** | **Raw material** | | **% w/w** | **% w/w** | **% w/w** |
| **A** | Tegin® M Pellets MB (Glyceryl Stearate) | | 0.5 | 0.5 | 0.5 |
| | Tego® Alkanol 1618 (Cetearyl Alcohol) | | 0.5 | 0.5 | 0.5 |
| | Tegosoft® XC MB (Phenoxyethyl Caprylate) | | 4.0 | 4.0 | 4.0 |
| | **Polyglycerol partial ester (PGE) Ex. 1** | | - | **1.5** | - |
| | LexFilm™ Sun Natural MB (Inolex, INCI: Capryloyl Glycerin/Sebacic Acid Copolymer) | | - | - | 1.5 |
| | Bis-ethylhexyloxyphenol Methoxyphenyl Triazine | | 3 | 3 | 3 |
| | Butyl Methoxy Dibenzoylmethane | | 2 | 2 | 2 |
| | Ethylhexyl Salicylate | | 3 | 3 | 3 |
| | Octrocrylene | | 8 | 8 | 8 |
| | Homosalate | | 3 | 3 | 3 |
| | Ethylhexyl Triazone | | 1.5 | 1.5 | 1.5 |
| | dermofeel® Toco 70 non-GMO (Tocopherol, Helianthus Annuus (Sunflower) Seed Oil) | | 0.2 | 0.2 | 0.2 |
| **B** | Potassium Cetyl Phosphate | | 1.5 | 1.5 | 1.5 |
| | Glycerin | | 2.5 | 2.5 | 2.5 |
| | Water | | to 100 | to 100 | to 100 |
| | Disodium EDTA | | 0.05 | 0.05 | 0.05 |

(continued)

| Phase | Raw material | V1 % w/w | V2* % w/w | V3 % w/w |
|---|---|---|---|---|
| C | Tegosoft® XC MB (Phenoxyethyl Caprylate) | 0.3 | 0.3 | 0.3 |
| | Tego Carbomer 341 ER (Carbomer) | 0.3 | 0.3 | 0.3 |
| | NaOH 10% soln. in water | 0.3 | 0.3 | 0.3 |
| D | Verstatil® PC Phenoxyethanol, Caprylyl Glycol | 1 | 1 | 1 |

Table 5b: Composition of the sun protection formulations for the sensory properties test

| Phase | Raw material | V4* % w/w | V5 % w/w | V6* % w/w | V7 % w/w |
|---|---|---|---|---|---|
| A | Tego® Care PBS 6 MB (Polyglyceryl-6 Stearate, Polyglyceryl-6 Behenate) | 3 | 3 | 3 | 3 |
| | Tegin® M Pellets MB (Glyceryl Stearate) | 0.5 | 0.5 | 0.5 | 0.5 |
| | Tego® Alkanol 1618 (Cetearyl Alcohol) | 0.5 | 0.5 | 0.5 | 0.5 |
| | dermofeel® sensolv MB (Isoamyl Laurate) | 4.3 | 4.3 | 4.3 | 4.3 |
| | **Polyglycerol partial ester (PGE) Ex. 1** | **1.5** | - | **1.5** | - |
| | Antaron™ V-220 (VP/Eicosene Copolymer, Ashland) | - | 1.5 | - | 1.5 |
| | Bis-ethylhexyloxyphenol Methoxyphenyl Triazine | 3 | 3 | 3 | 3 |
| | Butyl Methoxy Dibenzoylmethane | 2 | 2 | 2 | 2 |
| | Ethylhexyl Salicylate | 3 | 3 | 3 | 3 |
| | Octrocrylene | 8 | 8 | 8 | 8 |
| | Homosalate | 3 | 3 | 3 | 3 |
| | Ethylhexyl Triazone | 1.5 | 1.5 | 1.5 | 1.5 |
| | dermofeel® Toco 70 non-GMO (Tocopherol, Helianthus Annuus (Sunflower) Seed Oil) | 0.2 | 0.2 | 0.2 | 0.2 |
| B | Glycerin | 2 | 2 | 2 | 2 |
| | Water | to 100 | to 100 | to 100 | to 100 |
| | EDTA | 0.05 | 0.05 | 0.05 | 0.05 |
| C | Xanthan Gum (Keltrol CG-SFT, CP Kelco) | 0.3 | 0.3 | 0.3 | 0.3 |
| | dermofeel® sensolv MB (Isoamyl Laurate) | 0.3 | 0.3 | - | - |
| | Tego Carbomer 341 ER (Carbomer) | 0.3 | 0.3 | - | - |
| | NaOH 10% soln. in water | 0.3 | 0.3 | - | - |
| D | Verstatil® PC (Phenoxyethanol, Caprylyl Glycol) | 1 | 1 | 1 | 1 |

With regard to the use properties of the polyglycerol partial esters (PGEs) of the present invention, there were differences in sensory properties compared to the blank formulation without film former and compared to a likewise natural film former of the prior art that result in an improved skin feel during and after application. The results are collated in Table 6. Compared to the blank formulation V1, in V2 the polyglycerol partial ester (PGE) of the present invention brought about a significant increase in "velvety-silkiness" skin feel during and 5 min after application. The "velvety-silkiness" during application and after 5 min is also highest compared to the natural film former counterpart in V3. The polyglycerol partial ester (PGE) of the present invention is likewise able to achieve higher spreadability and reduced tackiness after 5 min compared to the blank formulation and to the formulation comprising the natural film former counterpart. The reduced tackiness is of particular interest in sun protection formulations, because the UV filters are generally associated with high tackiness.

Both in a test formulation based on a carbomer thickener (V4) and with a natural thickener (V6), the polyglycerol partial ester (PGE) of the present invention exhibits increased "velvety-silkiness" during application and after 5 min compared to a synthetic, non-biodegradable film former (V5 and V7 respectively). Here too, reduced "tackiness" after 5 min was observed in both thickener systems. The absorption during application and after 5 min is higher with the polyglycerol partial ester (PGE) of the present invention in both thickener systems.

Table 6: Results of the sensory properties tests

|  | Spreadability | Absorption | Velvety-silky | Absorption after 5 min | Velvety-silky after 5 min | Tackiness after 5 min |
|---|---|---|---|---|---|---|
| V1 | 6 | 6 | 1 | 6 | 1 | 4 |
| V2* | 8 | 8 | 3 | 8 | 3 | 2 |
| V3 | 5 | 6 | 0 | 6 | 1 | 5 |
|  |  |  |  |  |  |  |
| V4* | 7 | 5 | 2 | 8 | 3 | 2 |
| V5 | 5 | 4 | 0 | 6 | 1 | 4 |
|  |  |  |  |  |  |  |
| V6* | 6 | 7 | 1 | 8 | 2 | 1 |
| V7 | 5 | 5 | 0 | 7 | 0 | 4 |

*Further formulation examples*

The following formulation examples demonstrate the usability of the inventive polyglycerol partial esters in cosmetic emulsions by way of example and do not limit the subject matter of the invention.
All percent values are unless otherwise stated percentages by weight. Production and homogenization steps are carried out according to usual methods.
If necessary, the pH is adjusted with acids or bases, this being noted accordingly in the formulation. Since the amount of acid or base needed can depend on the batches of the other ingredients, it is in the examples here often entered as q.s. (= quantum satis). It is also necessary to adjust to different pH values depending on the preservative used. Customary pH values that were used and adjusted to here in the example formulations are in the range from pH 3.5 to 8.0.
The example recipes listed hereinbelow were respectively produced with each of the inventive PGEs from examples 1 to 10.

Fun in the Sun SPF 30 Spray

| Phase | Raw material | % w/w | % w/w |
|---|---|---|---|
| A | Tego® Care PBS 6 MB (Polyglyceryl-6 Stearate, Polyglyceryl-6 Behenate) | 3.00 | 3.00 |
|  | dermofeel® sensolv MB (Isoamyl Laurate) | 3.00 | 3.00 |
|  | Bis-Ethylhexyloxyphenol Methoxyphenyl Triazine (Tinosorb S, BASF) | 3.00 | 3.00 |
|  | Butyl Methoxydibenzoylmethane | 2.00 | 2.00 |
|  | Homosalate | 4.00 | 4.00 |
|  | Ethylhexyl Salicylate | 4.00 | 4.00 |
|  | Octocrylene | 4.00 | 4.00 |
|  | **Polyglycerol partial ester (PGE)** | **0.50** | **1.50** |
|  | VP/Eicosene Copolymer (Antaron V-220, Ashland) | 0.50 | |
|  | Tego® Feel C 10 (Cellulose) | 1.00 | 1.00 |
| B | Water | to 100 | to 100 |

20

(continued)

| Phase | Raw material | % w/w | % w/w |
|---|---|---|---|
| | Gellan Gum (Kelcogel CG-HA, CP Kelco) | 0.03 | 0.03 |
| | EDTA | 0.05 | 0.05 |
| | Glycerin | 3.00 | 3.00 |
| | Acrylates/C10-30 Alkyl Acrylate Crosspolymer | 0.20 | 0.20 |
| C | Phenylbenzimidazole Sulfonic Acid | 2.00 | 2.00 |
| | Tromethamine | 0.88 | 0.88 |
| | Water | 7.12 | 7.12 |
| D | Tromethamine (Trisaminomethane, 30% in water) | q.s. | q.s. |
| E | dermofeel® OMP (Methylpropanediol, Caprylyl Glycol, Phenylpropanol) | 3.00 | 3.00 |

Oil Release Sun Care Lotion SPF 50

| Phase | Raw material | % w/w | % w/w |
|---|---|---|---|
| A | Tego® Care PBS 6 MB (Polyglyceryl-6 Stearate, Polyglyceryl-6 Behenate) | 3.00 | 3.00 |
| | Tegosoft® XC (Phenoxyethyl Caprylate) | 15.00 | 15.00 |
| | Tegosoft® DC MB (Decyl Cocoate) | 10.00 | 10.00 |
| | Tegosoft® CT (Caprylic/Capric Triglyceride) | 10.00 | 10.00 |
| | **Polyglycerol partial ester (PGE)** | **1.50** | **2.50** |
| | Diethylamino Hydroxybenzoyl Hexyl Benzoate (Uvinul A plus, BASF) | 10.00 | 10.00 |
| | Ethylhexyl Methoxycinnamate | 10.00 | 10.00 |
| | Bis-Ethylhexyloxyphenol Methoxyphenyl Triazine (Tinosorb S, BASF) | 5.00 | 5.00 |
| | Ethylhexyl Salicylate | 5.00 | 5.00 |
| | Homosalate | 5.00 | 5.00 |
| | Tego® Feel C 10 (Cellulose) | 1.00 | - |
| | Dimethicone Crosspolymer | - | 0.50 |
| B | Water | to 100 | to 100 |
| | Glycerin | 3.00 | 3.00 |
| C | Verstatil® TBO (Triethyl Citrate; Caprylyl Glycol; Benzoic Acid) | 1.20 | 1.20 |
| D | Citric Acid (10% in water) | q.s. to pH 5.2 | q.s. to pH 5.2 |

Alcoholic Sun Care Spray SPF 30

| Phase | Raw material | % w/w | % w/w |
|---|---|---|---|
| A | Tegosoft® CT (Caprylic/Capric Triglyceride) | 20.00 | 20.00 |
| | Tegosoft® TN (C12-15 Alkyl Benzoate) | 8.00 | 8.00 |
| | Tegosoft® APM (PPG-3 Myristyl Ether) | 8.00 | 8.00 |
| | **Polyglycerol partial ester (PGE)** | **3.00** | **1.80** |

(continued)

| Phase | Raw material | % w/w | % w/w |
|---|---|---|---|
| | VP/Hexadecene Copolymer (Antaron V-216, Ashland) | - | 0.70 |
| | Tego® Xymenynic (Caprylic/Capric Triglyceride; Xymenynic Acid) | 2.00 | 2.00 |
| | Octocrylene | 6.80 | 6.80 |
| | Ethylhexyl Salicylate | 2.50 | 2.50 |
| | Butyl Methoxydibenzoylmethane | 5.00 | 5.00 |
| | Bis-Ethylhexyloxyphenol Methoxyphenyl Triazine (Tinosorb S, BASF SE) | 3.00 | 3.00 |
| | Diethylhexyl Butamido Triazone | 1.20 | 1.20 |
| B | Alcohol | to 100 | to 100 |

Alcoholic Sun Care Spray SPF 30

| Phase | Raw material | % w/w | % w/w |
|---|---|---|---|
| A | Tegosoft® CT (Caprylic/Capric Triglyceride) | 20.00 | 20.00 |
| | dermofeel® sensolv MB (Isoamyl Laurate) | 8.00 | 8.00 |
| | Tegosoft® APM (PPG-3 Myristyl Ether) | 8.00 | 8.00 |
| | **Polyglycerol partial ester (PGE)** | **3.00** | **1.80** |
| | VP/Hexadecene Copolymer (Antaron V-216, Ashland) | - | 0.70 |
| | Tego® Xymenynic (Caprylic/Capric Triglyceride; Xymenynic Acid) | 2.00 | 2.00 |
| | Octocrylene | 6.80 | 6.80 |
| | Ethylhexyl Salicylate | 2.50 | 2.50 |
| | Butyl Methoxydibenzoylmethane | 5.00 | 5.00 |
| | Bis-Ethylhexyloxyphenol Methoxyphenyl Triazine (Tinosorb S, BASF SE) | 3.00 | 3.00 |
| | Diethylhexyl Butamido Triazone | 1.20 | 1.20 |
| B | Alcohol | to 100 | to 100 |

Transparent Sun Care Spray SPF 25

| Phase | Raw material | % w/w |
|---|---|---|
| A | Tegosoft® P (Isopropyl Palmitate) | 12.00 |
| | **Polyglycerol partial ester (PGE)** | **1.00** |
| | Tegosoft® APM (PPG-3 Myristyl Ether) | 3.00 |
| | Tegosoft® GC (PEG-7 Glyceryl Cocoate) | 1.50 |
| | Tego® Turmerone (Curcuma Longa (Turmeric) Root Extract) | 0.50 |
| | Prunus Amygdalus Dulcis (Sweet Almond) Oil | 2.00 |
| | Dimethicone | 5.00 |
| | Homosalate | 10.00 |
| | Octocrylene | 9.40 |

(continued)

| Phase | Raw material | % w/w |
|---|---|---|
| | Ethylhexyl Salicylate | 4.50 |
| | Butyl Methoxydibenzoylmethane | 3.00 |
| | Tocopheryl Acetate | 0.50 |
| B | Denat. Alcohol | to 100 |

Water Resistant Aerosol Spray SPF 30

| Phase | Raw material | % w/w | % w/w |
|---|---|---|---|
| A | Denat. Alcohol | to 100 | to 100 |
| | Polyester-10 (and) Propylene Glycol Dibenzoate | 1.50 | 1.50 |
| | **Polyglycerol partial ester (PGE)** | **0.75** | **3.00** |
| | Tego® SP 13 Sun Up MB (Poly C10-30 Alkyl Acrylate) | 0.90 | - |
| | Benzophenone-3 | 5.00 | 5.00 |
| | Ethylhexyl Salicylate | 5.00 | 5.00 |
| | Ethylhexyl Methoxycinnamate | 7.50 | 7.50 |
| | Homosalate | 10.00 | 10.00 |
| | Butyl Methoxydibenzoylmethane | 2.00 | 2.00 |
| | Tegosoft® M (Isopropyl Myristate) | 4.00 | 4.00 |
| B | Drivosol® 27D60 (Butane; Propane; Isobutane) | 10.00 | 20.00 |

Water Resistant Aerosol Spray SPF 30

| Phase | Raw material | % w/w | % w/w |
|---|---|---|---|
| A | Denat. Alcohol | to 100 | to 100 |
| | Polyester-10 (and) Propylene Glycol Dibenzoate | 1.50 | 1.50 |
| | **Polyglycerol partial ester (PGE)** | **0.75** | **3.00** |
| | Tego® SP 13 Sun Up MB (Poly C10-30 Alkyl Acrylate) | 0.90 | - |
| | Benzophenone-3 | 5.00 | 5.00 |
| | Ethylhexyl Salicylate | 5.00 | 5.00 |
| | Ethylhexyl Methoxycinnamate | 7.50 | 7.50 |
| | Homosalate | 10.00 | 10.00 |
| | Butyl Methoxydibenzoylmethane | 2.00 | 2.00 |
| | dermofeel® sensolv MB (Isoamyl Laurate) | 6.00 | 6.00 |
| B | Drivosol® 27D60 (Butane; Propane; Isobutane) | 10.00 | 20.00 |

Light O/W Sun Care Lotion

| Phase | Raw material | % w/w | % w/w | % w/w | % w/w |
|---|---|---|---|---|---|
| A | Axol® C 62 Pellets MB (Glyceryl Stearate Citrate) | 2.50 | 2.50 | 0.50 | 2.50 |
| | Glyceryl Stearate SE | - | 1.00 | 4.00 | 1.00 |
| | Cetearyl Alcohol | 1.00 | 1.00 | - | 1.00 |
| | Glyceryl Stearate | - | - | 1.20 | - |
| | Tegosoft® CT (Caprylic/Capric Triglyceride) | 8.00 | 2.00 | - | 2.00 |
| | Tegosoft® TN (C12-15 Alkyl Benzoate) | - | 6.00 | 7.00 | 6.00 |
| | Diisopropyl Adipate | 1.00 | 1.00 | 1.00 | 1.00 |
| | **Polyglycerol partial ester (PGE)** | **2.00** | **2.00** | **2.00** | **1.50** |
| | Butyloctyl Salicylate (HallBrite BHB, The HallStar Company) | 2.00 | 2.00 | 2.00 | 2.00 |
| | Tocopheryl Acetate | 0.20 | 0.20 | 0.20 | 0.20 |
| | Dimethicone (5 mPas) | 1.00 | - | - | 0.50 |
| | Tapioca Starch | - | 1.00 | - | - |
| | Hydroxyethyl Acrylate/Sodium Acryloyldimethyl Taurate Copolymer | | | 1.20 | - |
| | Triacontanyl PVP (Antaron WP-660, Ashland) | - | - | - | 1.00 |
| | Butyl Methoxydibenzoylmethane | 1.50 | 1.50 | 1.50 | 1.50 |
| | Diethylamino Hydroxybenzoyl Hexyl Benzoate (Uvinul A Plus, BASF) | 4.00 | 4.00 | 4.00 | 4.00 |
| | Ethylhexyl Triazone | 2.00 | 2.00 | 2.00 | 2.00 |
| | Ethylhexyl Salicylate | 5.00 | 5.00 | 5.00 | 5.00 |
| | Octocrylene | 8.00 | 8.00 | 8.00 | 8.00 |
| | Ethylhexyl Methoxycrylene | 1.50 | 1.50 | 1.50 | 1.50 |
| B | Titanium Dioxide; Trimethoxycaprylylsilane | 1.00 | 1.00 | 1.00 | 1.00 |
| C | Glycerin | 3.20 | 3.20 | 3.20 | 3.20 |
| | Propanediol | 2.50 | 2.50 | 2.50 | 2.50 |
| | EDTA | 0.02 | 0.02 | 0.02 | 0.02 |
| | Water | to 100 | to 100 | to 100 | to 100 |
| D | Acrylates/C10-30 Alkyl Acrylate Crosspolymer | 0.10 | 0.10 | 0.10 | 0.10 |
| | Tegosoft® CT (Caprylic/Capric Triglyceride) | 0.90 | 0.90 | 0.90 | 0.90 |
| E | Sodium Hydroxide (10% in water) | q.s. | q.s. | q.s. | q.s. |
| Z | Preservative, Perfume | q.s. | q.s. | q.s. | q.s. |

Dry Touch Hand Moisturizing Cream

| Phase | Raw material | % w/w | % w/w | % w/w |
|---|---|---|---|---|
| | Varisoft® TA 100 (Distearyldimonium Chloride) | 3.00 | 3.00 | - |
| | Tego® Care Eco CAT MB (Distearoylisopropyl Dimonium Methosulfate; Stearyl Alcohol) | - | - | 4.50 |
| | Stearyl Alcohol | 1.00 | 1.00 | 1.00 |
| | Tegin® M Pellets MB (Glyceryl Stearate) | 1.00 | 1.00 | 2.00 |
| | Tegosoft® SH (Stearyl Heptanoate) | 1.00 | 1.00 | 1.00 |

(continued)

| Phase | Raw material | % w/w | % w/w | % w/w |
|---|---|---|---|---|
| A | Tegosoft® P (Isopropyl Palmitate) | 5.00 | 5.00 | 5.00 |
| | **Polyglycerol partial ester (PGE)** | **1.50** | **2.00** | **2.00** |
| | Triacontanyl PVP (Antaron WP-660, Ashland) | 0.50 | - | - |
| | Diethylamino Hydroxybenzoyl Hexyl Benzoate (Uvinul A plus, BASF) | 3.00 | 3.00 | 3.00 |
| | Ethylhexyl Salicylate | 3.00 | 3.00 | 3.00 |
| | Ethylhexyl Methoxycinnamate | 5.00 | 5.00 | 5.00 |
| | Tocopheryl Acetate | 0.50 | 0.50 | 0.50 |
| | Tego® Feel C 10 (Cellulose) | 1.00 | - | 1.00 |
| B | Water | to 100 | to 100 | to 100 |
| | Glycerin | 3.00 | 3.00 | 3.00 |
| C | Tego® Natural Betaine (Betaine) | 2.00 | 2.00 | 2.00 |
| | Water | 2.00 | 2.00 | 2.00 |
| D | Phenoxyethanol, Ethylhexylglycerin (Euxyl PE 9010, Schülke & Mayr GmbH) | 0.70 | 0.70 | 0.70 |

Age Defense BB Cream SPF 15

| Phase | Raw material | % w/w | % w/w | % w/w |
|---|---|---|---|---|
| A | C14-22 Alcohols; C12-20 Alkyl Glucoside | 4.50 | - | 3.50 |
| | Cetearyl Alcohol; Cetearyl Glucoside | - | 5.20 | - |
| | Potassium Cetyl Phosphate | 0.80 | - | - |
| | Sodium Stearoyl Glutamate | - | 1.10 | 2.00 |
| | Glyceryl Stearate | 1.00 | 1.00 | 1.00 |
| | Stearic Acid | 0.50 | 0.50 | 0.50 |
| | Stearyl Alcohol | 1.00 | 1.00 | 1.00 |
| | Butylene Glycol Dicaprylate/Dicaprate | 5.00 | 2.00 | 5.50 |
| | Tegosoft® DEC (Diethylhexyl Carbonate) | 1.90 | 4.90 | 1.40 |
| | Capryloyl Glycerin/Sebacic Acid Copolymer (LexFilm Sun Natural MB, Inolex Inc.) | 1.50 | - | - |
| | **Polyglycerol partial ester (PGE)** | **1.50** | **3.00** | **3.00** |
| | HyaCare® Filler CL (Water; Ethylhexyl Stearate; Sodium Hyaluronate Crosspolymer; Polyglyceryl-4 Diisostearate/ Polyhydroxystearate/ Sebacate; Sodium Isostearate) | 2.00 | 2.00 | 2.00 |
| | Phytosphingosine | 0.10 | 0.10 | 0.10 |
| | Ethylhexyl Methoxycinnamate | 5.00 | 5.00 | 5.00 |
| | Diethylamino Hydroxybenzoyl Hexyl Benzoate | 3.00 | 3.00 | 3.00 |
| B | Titanium Dioxide (Hombitan AC 360, Sachtleben) | 3.00 | 3.00 | 3.00 |
| | Talc (Micro Talc IT Extra-AW, Mondo Minerals B.V.) | 2.00 | 2.00 | 2.00 |
| | Unipure Yellow LC 182 | 0.36 | 0.36 | 0.36 |

(continued)

| Phase | Raw material | % w/w | % w/w | % w/w |
|---|---|---|---|---|
| | Unipure Red LC 381 | 0.12 | 0.12 | 0.12 |
| | Unipure Black LC 989 | 0.08 | 0.08 | 0.08 |
| | Tegosoft® AC MB (Isoamyl Cocoate) | 4.40 | 4.40 | 4.40 |
| | Tegosoft® CT (Caprylic/Capric Triglyceride) | 4.00 | 4.00 | 4.00 |
| | Polymethyl Methacrylate (Sepimat P, Seppic) | 3.00 | 3.00 | 3.00 |
| | Polyhydroxystearic Acid | 0.50 | 0.50 | - |
| C | Water | to 100 | to 100 | to 100 |
| | Glycerin | 3.00 | 3.00 | 3.00 |
| | HyaCare® 50 (Hydrolysed Hyaluronic Acid) | 0.10 | 0.10 | 0.10 |
| | Tego® Pep 4-17 (Tetrapeptide-21; Glycerin; Butylene Glycol; Water) | 2.00 | 2.00 | 2.00 |
| D | Alcohol | 3.00 | 3.00 | 3.00 |
| E | Preservative | q.s. | q.s. | q.s. |

Moisture Caring BB Cream SPF 15

| Phase | Raw material | % w/w | % w/w |
|---|---|---|---|
| A | Abil® EM 180 (Cetyl PEG/PPG-10/1 Dimethicone) | 2.00 | 2.00 |
| | Abil® Wax 9801 (Cetyl Dimethicone) | 2.00 | 2.00 |
| | Diethylamino Hydroxybenzoyl Hexyl Benzoate; Ethylhexyl Methoxycinnamate | 10.00 | 10.00 |
| | Dicaprylyl Carbonate | 2.00 | 2.00 |
| | **Polyglycerol partial ester (PGE)** | **0.50** | **1.50** |
| | Dimethicone (and) Trimethylsiloxysilicate | 3.00 | 3.00 |
| | HyaCare® Filler CL (Water; Ethylhexyl Stearate; Sodium Hyaluronate Crosspolymer; Polyglyceryl-4 Diisostearate/Polyhydroxystearate/ Sebacate; Sodium Isostearate) | 2.00 | 2.00 |
| | Phytosphingosine (Phytosphingosine) | 0.10 | 0.10 |
| | Hydrogenated Castor Oil | 0.50 | 0.50 |
| | Microcrystalline Wax | 0.50 | 0.50 |
| B | Talc (Micro Talc IT Extra-AW, Mondo Minerals B.V.) | 2.00 | 2.00 |
| | Titanium Dioxide (Hombitan AC 360, Sachtleben) | 4.00 | 4.00 |
| | Unipure Yellow LC 182 | 0.36 | 0.36 |
| | Unipure Red LC 381 | 0.12 | 0.12 |
| | Unipure Black LC 989 | 0.08 | 0.08 |
| | Aerosil® R 972 (Silica Dimethylsilylate) | 0.50 | - |
| | Tegosoft® OS (Ethylhexyl Stearate) | 5.00 | 5.00 |
| | Tegosoft® DEC (Diethylhexyl Carbonate) | 6.00 | 6.00 |
| C | Water | to 100 | to 100 |
| | HyaCare® (Sodium Hyaluronate) | 0.05 | 0.05 |

(continued)

| Phase | Raw material | % w/w | % w/w |
|---|---|---|---|
| | Glycerin | 5.00 | 5.00 |
| | Sodium Chloride | 0.80 | 0.80 |
| D | Phenoxyethanol; Ethylhexylglycerin (Euxyl PE 9010, Schülke & Mayr) | 0.70 | 0.70 |

Anhydrous stick

| Phase | Raw material | % w/w |
|---|---|---|
| | dermofeel® viscolid MB (Hydrogenated Vegetable Oil) | 10.00 |
| | Tego® SP 13 Sun Up (Poly C10-30 Alkyl Acrylate) | 5.00 |
| | **Polyglycerol partial ester (PGE)** | **10.00** |
| | Tegosoft® CT (Caprylic/Capric Triglyceride) | 30.00 |
| | Isopropyl Palmitate | 15.00 |
| | Ethylhexyl Methoxycinnamate | 7.50 |
| | Octyldodecanol | to 100 |

O/W Sun Protect & Bronze

| Phase | Raw material | % w/w | % w/w | % w/w | % w/w |
|---|---|---|---|---|---|
| | Glyceryl Stearate Citrate | 2.00 | 2.00 | 2.00 | 2.00 |
| | Tego® Alkanol 1618 (Cetearyl Alcohol) | 1.50 | 1.50 | 1.50 | 1.50 |
| | Tegosoft® DC MB (Decyl Cocoate) | 8.00 | 8.00 | 8.00 | 8.00 |
| | Tegosoft® XC MB (Phenoxyethyl Caprylate) | 4.00 | 4.00 | 4.00 | 4.00 |
| | Cera Alba (Beeswax 8104, KahlWax) | 0.50 | - | - | - |
| | **Polyglycerol partial ester (PGE)** | **0.50** | **2.00** | **1.10** | **1.30** |
| | Styrene/Acrylates Copolymer (Dermacryl E, Nouryon) | - | - | 1.00 | - |
| | Acrylates/Octylacrylamide Copolymer (Dermacryl 79, Nouryon) | | | | 0.8 |
| A | Titanium Dioxide, Trimethoxycaprylylsilane | 1.50 | 1.50 | 1.50 | 1.50 |
| | Dimethicone (5 cSt) | 3.00 | - | - | - |
| | Talc | - | 1.20 | 1.20 | 1.20 |
| | Octocrylene | 9.00 | 9.00 | 9.00 | 9.00 |
| | Ethylhexyl Salicylate | 4.50 | 4.50 | 4.50 | 4.50 |
| | Butyl Methoxydibenzoylmethane | 2.00 | 2.00 | 2.00 | 2.00 |
| | Tocopheryl Acetate | 0.50 | 0.50 | 0.50 | 0.50 |
| | Xanthan Gum (Keltrol CG-SFT, CP Kelco) | 0.50 | 0.50 | 0.50 | 0.50 |
| B | Water | to 100 | to 100 | to 100 | to 100 |
| | Glycerin | 3.00 | 3.00 | 3.00 | 3.00 |
| | Panthenol | 1.00 | 1.00 | 1.00 | 1.00 |

(continued)

| Phase | Raw material | % w/w | % w/w | % w/w | % w/w |
|---|---|---|---|---|---|
| C | Acrylates/C10-30 Alkyl Acrylate Crosspolymer | 0.10 | 0.10 | 0.10 | 0.10 |
| | Tegosoft® TN (C12-15 Alkyl Benzoate) | 0.40 | 0.40 | 0.40 | 0.40 |
| D | Sodium Hydroxide (10% in water) | q.s. | q.s. | q.s. | q.s. |
| E | Butylene Glycol; Acetyl-L-Tyrosine; Hydrolysed Vegetable Protein; Adenosine Triphosphate; Riboflavin (Unipertan VEG-2001) | 0.80 | 0.80 | 0.80 | 0.80 |
| Z | Preservative, Perfume | q.s. | q.s. | q.s. | q.s. |

Sun Care Foam SPF 50

| Phase | Raw material | % w/w | % w/w | % w/w |
|---|---|---|---|---|
| A | Tego® Care PBS 6 MB (Polyglyceryl-6 Stearate, Polyglyceryl-6 Behenate) | 2.00 | 2.00 | 2.00 |
| | Tegosoft® AC MB (Isoamyl Cocoate) | 5.00 | 5.00 | 5.00 |
| | dermofeel® viscolid MB (Hydrogenated Vegetable Oil) | 0.50 | 0.50 | 0.50 |
| | **Polyglycerol partial ester (PGE)** | **0.50** | **0.50** | **0.50** |
| | Bis-Ethylhexyloxyphenol Methoxyphenyl Triazine | 4.50 | 4.50 | 4.50 |
| | Butyl Methoxydibenzoylmethane | 3.00 | 3.00 | 3.00 |
| | Octocrylene | 8.00 | 8.00 | 8.00 |
| | Homomenthyl Salicylate | 6.00 | 6.00 | 6.00 |
| | Ethylhexyl Salicylate | 2.50 | 2.50 | 2.50 |
| | Tego® Feel C 10 (Cellulose) | 2.00 | - | - |
| | Methyl Methacrylate Crosspolymer | - | 1.50 | - |
| | Aluminium Starch Octenylsuccinate | - | - | 1.70 |
| B | Water | to 100 | to 100 | to 100 |
| | Glycerin | 2.00 | 2.00 | 2.00 |
| | EDTA Disodium | 0.05 | 0.05 | 0.05 |
| C | C12-15 Alkyl Benzoate | 0.80 | 0.80 | 0.80 |
| | Carbomer | 0.20 | 0.20 | 0.20 |
| | Xanthan Gum (Keltrol CG-SFT, CP Kelco) | 0.20 | 0.20 | 0.20 |
| D | Water | 11.68 | 11.68 | 11.68 |
| | Phenylbenzimidazole Sulfonic Acid | 3.00 | 3.00 | 3.00 |
| | Tromethamine | 1.32 | 1.32 | 1.32 |
| E | Phenoxyethanol, Ethylhexylglycerin (Euxyl PE 9010, Schülke & Mayr GmbH) | 0.70 | 0.70 | 0.70 |
| | Tego® Betain 810 (Capryl/Capramidopropyl Betaine) | 1.00 | 1.00 | 1.00 |
| | Rewoteric® AM C (Sodium Cocoamphoacetate) | 1.00 | 1.00 | 1.00 |
| F | Tromethamine (30% in water) | q.s. | q.s. | q.s. |

W/O Sun Protection Shake-Shake

| Phase | Raw material | % w/w |
|---|---|---|
| A | Abil® EM 90 (Cetyl PEG/PPG-10/1 Dimethicone) | 1.00 |
| | Tegosoft® CT (Caprylic/Capric Triglyceride) | 11.00 |
| | Dicaprylyl Carbonate | 2.50 |
| | Dimethicone (5 cSt) | 12.00 |
| | Octocrylene | 6.00 |
| | Ethylhexyl Salicylate | 2.00 |
| | Butyl Methoxydibenzoylmethane | 0.80 |
| | Bis-Ethylhexyloxyphenol Methoxyphenyl Triazine (Tinosorb S, BASF SE) | 1.50 |
| | Titanium Dioxide, Trimethoxycaprylylsilane | 2.00 |
| | **Polyglycerol partial ester (PGE)** | **0.50** |
| B | Water | 4.43 |
| | Phenylbenzimidazole Sulfonic Acid (Eusolex 232, Merck KGaA) | 0.50 |
| | Sodium Hydroxide | 0.07 |
| C | Distarch Phosphate (MAIS PO4 PH "B", Agrana Stärke GmbH) | 1.50 |
| | Alcohol | 1.00 |
| | Glycerin | 6.00 |
| | Water | to 100 |
| Z | Preservative, Perfume | q.s. |

Light Sun Care W/O Shake-Shake SPF30 PA+++

| Phase | Raw material | % w/w |
|---|---|---|
| A | Abil® EM 90 (Cetyl PEG/PPG-10/1 Dimethicone) | 1.50 |
| | Tegosoft® DEC (Diethylhexyl Carbonate) | 5.00 |
| | Dimethicone (2 cSt) | 20.00 |
| | Tegosoft® CT (Caprylic/Capric Triglyceride) | 5.00 |
| | C12-15 Alkyl Benzoate; Titanium Dioxide; PEG-30 Dipolyhydroxystearate; Silica; Dimethicone (T-lique AB50Si, Uni-Powder) | 2.40 |
| | Zinc Oxide (ZnO-NAS, Sunjin) | 8.00 |
| | Abil® Wax 9801 (Cetyl Dimethicone) | 2.00 |
| | Talc | 2.00 |
| | Polymethylsilsesquioxane (Gransil PSQ, Grant) | 3.00 |
| | Vinyl Dimethicone/Methicone Silsesquioxane Crosspolymer (KSP-100, ShinEtsu) | 1.00 |
| | **Polyglycerol partial ester (PGE)** | **2.00** |
| | Diethylamino Hydroxybenzoyl Hexyl Benzoate | 2.00 |
| | Ethylhexyl Methoxycinnamate | 8.00 |
| | Ethylhexyl Salicylate | 4.00 |
| | Bis-Ethylhexyloxyphenol Methoxyphenyl Triazine (Escalol S, Ashland) | 0.50 |

(continued)

| Phase | Raw material | % w/w |
|-------|--------------|-------|
| B | Water | to 100 |
| | Glycerin | 3.00 |
| | Sodium Chloride | 1.00 |
| C | Phenoxyethanol, Ethylhexylglycerin (Euxyl PE 9010, Schülke & Mayr GmbH) | 0.70 |

W/O Organic shake-shake SPF50+ PA++++

| Phase | Raw material | % w/w |
|-------|--------------|-------|
| A | Abil® EM 90 (Cetyl PEG/PPG-10/1 Dimethicone) | 1.50 |
| | Tegosoft® DEC (Diethylhexyl Carbonate) | 7.00 |
| | Dimethicone (5 cSt) | 5.00 |
| | Cyclopentasiloxane; Cyclohexasiloxane | 5.00 |
| | Tegosoft® P (Isopropyl Palmitate) | 3.00 |
| | **Polyglycerol partial ester (PGE)** | **0.50** |
| | C12-15 Alkyl Benzoate; Titanium Dioxide; PEG-30 Dipolyhydroxystearate; Silica; Dimethicone (T-lique AB50Si, Uni-Powder) | 15.60 |
| | Abil® Wax 9801 (Cetyl Dimethicone) | 2.00 |
| | Bis-Ethylhexyloxyphenol Methoxyphenyl Triazine (Escalol S, Ashland) | 1.00 |
| | Diethylamino Hydroxybenzoyl Hexyl Benzoate (Uvinul A plus Granular, BASF) | 1.50 |
| | Octocrylene | 2.00 |
| | Ethylhexyl Methoxycinnamate | 7.00 |
| | Ethylhexyl Salicylate | 4.00 |
| | Zinc Oxide (ZnO-NAS, Sunjin) | 14.40 |
| B | Water | to 100 |
| | Butylene Glycol | 3.00 |
| | Sodium Chloride | 1.50 |
| C | Phenoxyethanol, Ethylhexylglycerin (Euxyl PE 9010, Schülke & Mayr GmbH) | 0.70 |

Sun Care Cream SPF 30

| Phase | Raw material | % w/w | % w/w |
|-------|--------------|-------|-------|
| A | dermofeel® GSC SG (Glyceryl Stearate Citrate) | 2.00 | 2.00 |
| | Tegin® M Pellets MB (Glyceryl Stearate) | 0.50 | 0.50 |
| | Tego® Alkanol 1618 (Cetearyl Alcohol) | 0.50 | 0.50 |
| | dermofeel® sensolv MB (Isoamyl Laurate) | 4.50 | 4.50 |
| | Bis-Ethylhexyloxyphenol Methoxyphenyl Triazine (Tinosorb S, BASF) | 3.00 | 3.00 |
| | Butyl Methoxydibenzoylmethane | 2.00 | 2.00 |
| | Ethylhexyl Salicylate | 3.00 | 3.00 |

(continued)

| Phase | Raw material | % w/w | % w/w |
|---|---|---|---|
| | Octocrylene | 8.00 | 8.00 |
| | Homosalate | 3.00 | 3.00 |
| | Ethylhexyl Triazone | 1.50 | 1.50 |
| | **Polyglycerol partial ester (PGE)** | **1.50** | **1.50** |
| B | Water | to 100 | to 100 |
| | Glycerin | 3.00 | 3.00 |
| | EDTA | 0.05 | 0.05 |
| | Tego® Feel C 10 (Cellulose) | 2.00 | - |
| | Zea Mays (Corn) Starch | - | 2.00 |
| | 1,2-Hexanediol | 2.20 | 2.20 |
| C | Acrylates/C10-30 Alkyl Acrylate Crosspolymer | 0.20 | 0.20 |
| | dermofeel® sensolv MB (Isoamyl Laurate) | 0.80 | 0.80 |
| D | Sodium Hydroxide (10% in water) | q.s. | q.s. |
| E | Verstatil® PC (Phenoxyethanol, Caprylyl Glycol) | 1.00 | 1.00 |

On the go UV protection stick SPF 50

| Phase | Raw material | % w/w |
|---|---|---|
| A | Tegosoft® M (Isopropyl Myristate) | 8.00 |
| | Tegosoft® CT (Caprylic/Capric Triglyceride) | 7.50 |
| | Tegosoft® DEC (Diethylhexyl Carbonate) | 8.60 |
| | Titanium Dioxide, Silica, Dimethicone (Parsol TX, DSM) | 2.50 |
| B | Tegosoft® CR MB (Cetyl Ricinoleate) | 2.60 |
| | Tegosoft® MM MB (Myristyl Myristate) | 4.00 |
| | Diethylamino Hydroxybenzoyl Hexyl Benzoate | 8.00 |
| | Ethylhexyl Salicylate | 5.00 |
| | Ethylhexyl Methoxycinnamate | 10.00 |
| | Homosalate | 5.00 |
| | Bis-Ethylhexyloxyphenol Methoxyphenyl Triazine (Tinosorb S, BASF) | 3.50 |
| | Beeswax | 3.50 |
| | Polyglyceryl-3 Beeswax (Cera Bellina, Koster Keunen) | 6.00 |
| | Ozokerite (1899, KahlWax) | 3.50 |
| | Copernicia Cerifera (Carnauba) Wax (2442L, KahlWax) | 2.50 |
| | dermofeel® AP MB (Ascorbyl Palmitate) | 0.10 |
| | dermofeel® Toco 70 non GMO (Tocopherol, Helianthus Annuus (Sunflower) Seed Oil) | 0.20 |
| | Tego® SP 13-1 (Poly C10-30 Alkyl Acrylate) | 2.50 |
| | **Polyglycerol partial ester (PGE)** | **5.00** |
| C | Aerosil® 200 (Silica) | 2.00 |

EP 4 279 531 B1

(continued)

| Phase | Raw material | % w/w |
|---|---|---|
| D | Tego® Feel C 10 (Cellulose) | 10.00 |
| Z | Perfume | q.s. |

Transparent UV protection water spray SPF 30

| Phase | Raw material | % w/w |
|---|---|---|
| A | Tegosoft® DEC (Diethylhexyl Carbonate) | 15.00 |
| | Isododecane | 7.50 |
| | Tegosoft® P (Isopropyl Palmitate) | 5.00 |
| | dermofeel® sensolv MB (Isoamyl Laurate) | 5.00 |
| | Butyl Methoxydibenzoylmethane | 5.00 |
| | Bis-Ethylhexyloxyphenol Methoxyphenyl Triazine (Tinosorb S, BASF) | 2.40 |
| | Homosalate | 2.00 |
| | Ethylhexyl Salicylate | 2.00 |
| | Ethylhexyl Triazone | 1.70 |
| | dermofeel® Toco 70 non GMO (Tocopherol, Helianthus Annuus (Sunflower) Seed Oil) | 0.50 |
| | **Polyglycerol partial ester (PGE)** | **0.35** |
| B | Verstatil® PC (Phenoxyethanol, Caprylyl Glycol) | 1.00 |
| | Perfume | q.s. |
| C | Water | to 100 |
| | Glycerin | 3.00 |
| | Disodium EDTA | 0.10 |
| D | Water | 9.65 |
| | Phenylbenzimidazole Sulfonic Acid | 2.50 |
| | Sodium Hydroxide | 0.35 |
| E | Triethanolamine | q.s. to pH 7.1 |

Sun Care Cream SPF 25

| Phase | Raw material | % w/w | % w/w |
|---|---|---|---|
| A | Glyceryl Stearate | 0.50 | 0.50 |
| | Cetearyl Alcohol | 0.50 | - |
| | Cetyl Alcohol | - | 0.80 |
| | dermofeel® sensolv MB (Isoamyl Laurate) | 3.50 | 3.50 |
| | Butyl Methoxydibenzoylmethane | 3.00 | 3.00 |
| | Ethylhexyl Salicylate | 5.00 | 5.00 |
| | Octocrylene | 10.0 | 10.0 |

(continued)

| Phase | Raw material | % w/w | % w/w |
|---|---|---|---|
| | Homosalate | 10.0 | 10.0 |
| | **Polyglycerol partial ester (PGE)** | **1.50** | **1.50** |
| B | Potassium Cetyl Phosphate (Amphisol K, DSM Nutritional Products) | 1.50 | 1.50 |
| | Water | to 100 | to 100 |
| | Glycerin | 3.00 | 3.00 |
| | EDTA | 0.05 | 0.05 |
| C | Acrylates/C10-30 Alkyl Acrylate Crosspolymer | 0.20 | 0.20 |
| | dermofeel® sensolv MB (Isoamyl Laurate) | 0.80 | 0.80 |
| D | Sodium Hydroxide (10% in water) | 0.60 | 0.60 |
| E | Verstatil® PC (Phenoxyethanol, Caprylyl Glycol) | 1.00 | 1.00 |

Inorganic water-resistant O/W sunscreen SPF 20

| Phase | Raw material | % w/w | % w/w |
|---|---|---|---|
| A | Tego® Care PBS 6 MB (Polyglyceryl-6 Stearate; Polyglyceryl-6 Behenate) | 4.00 | 4.00 |
| | Tego® SP 13-1 (Poly C10-30 Alkyl Acrylate) | 1.50 | - |
| | Tegin® M Pellets MB (Glyceryl Stearate) | 0.20 | 0.20 |
| | Tego® Alkanol 1618 (Cetearyl Alcohol) | 0.20 | 0.20 |
| B | Zinc Oxide; Triethoxycaprylylsilane (Z-Cote HP-1, BASF) | 7.80 | 7.80 |
| | Titanium Dioxide, Silica, Dimethicone (Parsol TX, DSM) | 7.20 | 7.20 |
| | Tegosoft® DC MB (Decyl Cocoate) | 3.00 | 3.00 |
| | dermofeel® sensolv MB (Isoamyl Laurate) | 3.00 | 3.00 |
| | Tegosoft® CT (Caprylic/Capric Triglyceride) | 5.00 | 5.00 |
| | Tegosoft® DEC (Diethylhexyl Carbonate) | 5.00 | 5.00 |
| | Abil® 350 (Dimethicone) | 3.00 | - |
| | Abil® Wax 9840 (Cetyl Dimethicone) | 2.00 | - |
| | Zea Mays (Corn) Starch | - | 2.00 |
| | dermosoft® Octiol (Caprylyl Glycol) | 0.50 | 0.50 |
| | **Polyglycerol partial ester (PGE)** | **0.50** | **2.50** |
| C | Water | to 100 | to 100 |
| | Glycerin | 3.00 | 3.00 |
| | Xanthan Gum (Keltrol CG-SFT, CP Kelco) | 0.50 | 0.50 |
| D | dermosoft® Pentiol eco (Pentylene Glycol) | 3.00 | 3.00 |
| E | Citric Acid (50% in water) | 0.45 | 0.45 |

Feel the sun spray SPF 50

| Phase | Raw material | % w/w | % w/w |
|---|---|---|---|
| A | dermofeel® GSC SG (Glyceryl Stearate Citrate) | 2.50 | 2.50 |
| | Tegin® M Pellets MB (Glyceryl Stearate) | 0.25 | 0.25 |
| | Tego® Alkanol 1618 (Cetearyl Alcohol) | 0.25 | 0.25 |
| | dermofeel® sensolv MB (Isoamyl Laurate) | 4.00 | 6.00 |
| | Di(iso)pentyl terephthalate | 4.00 | - |
| | **Polyglycerol partial ester (PGE)** | **1.00** | **1.00** |
| | Bis-Ethylhexyloxyphenol Methoxyphenyl Triazine (Tinosorb S, BASF) | 6.00 | 6.00 |
| | Diethylamino Hydroxybenzoyl Hexyl Benzoate | 6.00 | 6.00 |
| | Ethylhexyl Salicylate | 5.00 | 5.00 |
| | Ethylhexyl Triazone | 2.00 | 2.00 |
| | dermofeel® Toco 70 non GMO (Tocopherol, Helianthus Annuus (Sunflower) Seed Oil) | 0.20 | 0.20 |
| | Titanium Dioxide; Aluminium Hydroxide; Stearic Acid (A15-TiO$_2$-ST-SA8, Kobo Products) | - | 1.50 |
| B | Water | to 100 | to 100 |
| | Glycerin | 3.00 | 3.00 |
| | Propylene Glycol | 2.00 | 2.00 |
| | EDTA | 0.05 | 0.05 |
| C | Xanthan Gum (Keltrol CG-SFT, CP Kelco) | 0.50 | 0.50 |
| D | Phenylbenzimidazole Sulfonic Acid | 3.00 | 3.00 |
| | Tromethamine (Trisaminomethane) | 1.32 | 1.32 |
| | Water | 10.68 | 10.68 |
| E | Verstatil® BOB (Benzyl Alcohol; Caprylyl Glycol; Benzoic Acid) | 1.20 | 1.20 |
| Z | Perfume | 0.10 | 0.10 |

Natural Sun Protection Stick

| Phase | Raw material | % w/w |
|---|---|---|
| A | Carthamus Tinctorius (Safflower) Seed Oil | 22.80 |
| | Butyrospermum Parkii Butter (shea butter) | 7.00 |
| | Hippophae Rhamnoides Fruit Oil | 1.00 |
| | dermofeel® sensolv MB (Isoamyl Laurate) | 8.00 |
| | Tegosoft® CT (Caprylic/Capric Triglyceride) | 2.00 |
| | dermofeel® viscolid MB (Hydrogenated Vegetable Oil) | 2.00 |
| | Tegosoft® DC MB (Decyl Cocoate) | 6.00 |
| | Tegosoft® OER MB (Oleyl Erucate) | 6.00 |
| | Cera Alba (Beeswax 8104, KahlWax) | 5.00 |
| | Helianthus Annuus Seed Cera, Ascorbyl Palmitate, Tocopherol (Sunflower Seed Wax 6607L, KahlWax) | 3.00 |
| | Euphorbia Cerifera Cera (Candelilla Wax 2039L, KahlWax) | 3.00 |

(continued)

| Phase | Raw material | % w/w |
|---|---|---|
| | **Polyglycerol partial ester (PGE)** | **8.00** |
| | dermofeel® TocoBalance (Tocopherol, Helianthus Annuus (Sunflower) Seed Oil) | 1.50 |
| | dermosoft® GMCY MB (Glyceryl Caprylate) | 1.00 |
| B | Distarch Phosphate (Corn P04 PH "B", Agrana) | 1.00 |
| | Tego® Feel C 10 (Cellulose) | 2.00 |
| | Zinc Oxide (Zano 10, EverZinc) | 20.00 |
| C | Perfume | 0.70 |

W/O Quick-Breaking Cream SPF 15

| Phase | Raw material | % w/w |
|---|---|---|
| A | Abil® EM 180 (Cetyl PEG/PPG-10/1 Dimethicone) | 0.80 |
| | Tegosoft® DEC (Diethylhexyl Carbonate) | 3.00 |
| | Tegosoft® CT (Caprylic/Capric Triglyceride) | 2.00 |
| | Dimethicone (5 cSt) | 3.00 |
| | **Polyglycerol partial ester (PGE)** | **1.00** |
| | Bis-Ethylhexyloxyphenol Methoxyphenyl Triazine (Tinosorb S, BASF) | 2.00 |
| | Diethylamino Hydroxybenzoyl Hexyl Benzoate | 2.00 |
| | 4-Methylbenzylidene Camphor | 4.00 |
| | Ethylhexyl Salicylate | 2.00 |
| | Verstatil® BOB (Benzyl Alcohol, Caprylyl Glycol, Benzoic Acid) | 1.00 |
| B | Water | to 100 |
| | Propylene Glycol | 5.00 |
| | Sodium Chloride | 1.00 |

W/O Sun Care Lotion SPF 8, Water Resistant

| Phase | Raw material | % w/w | % w/w | % w/w | % w/w |
|---|---|---|---|---|---|
| A | Isolan® GPS (Polyglyceryl-4 Diisostearate / Polyhydroxystearate / Sebacate) | 3.00 | - | - | - |
| | Polyglyceryl-3 Diisostearate | - | 3.50 | - | - |
| | Polyglyceryl-2 Dipolyhydroxystearate | - | - | 3.00 | - |
| | Polyglyceryl-3 Polyricinoleate | - | - | - | 3.00 |
| | Hydrogenated Castor Oil | 0.20 | 0.20 | 0.20 | 0.20 |
| | Microcrystalline Wax | 0.30 | 0.30 | 0.30 | 0.30 |
| | Tegosoft® CT (Caprylic/Capric Triglyceride) | 5.00 | 5.00 | 5.00 | 5.00 |
| | Tegosoft® DEC (Diethylhexyl Carbonate) | 3.00 | 3.00 | 3.00 | 3.00 |
| | Tegosoft® CR MB (Cetyl Ricinoleate) | 0.75 | 0.75 | 0.75 | 0.75 |

(continued)

| Phase | Raw material | % w/w | % w/w | % w/w | % w/w |
|---|---|---|---|---|---|
| | **Polyglycerol partial ester (PGE)** | **0.75** | **0.75** | **0.75** | **0.75** |
| | Triisostearin | 1.00 | 1.00 | 1.00 | 1.00 |
| | Ethylhexyl Methoxycinnamate | 5.00 | 5.00 | 5.00 | 5.00 |
| | Ethylhexyl Salicylate | 4.00 | 4.00 | 4.00 | 4.00 |
| | Menthyl Anthranilate | 4.00 | 4.00 | 4.00 | 4.00 |
| B | Tego® Cosmo C 100 (Creatine) | 0.50 | 0.50 | 0.50 | 0.50 |
| | Glycerin | 3.00 | 3.00 | 3.00 | 3.00 |
| | Magnesium Sulfate Heptahydrate | 1.50 | 1.50 | 1.50 | 1.50 |
| | Water | to 100 | to 100 | to 100 | to 100 |
| Z | Preservative, Perfume | q.s. | q.s. | q.s. | q.s. |

W/O Sun Care Lotion, Water resistant

| Phase | Raw material | % w/w |
|---|---|---|
| A | Isolan® 17 MB (Polyglyceryl-4 Diisostearate/Polyhydroxystearate/Sebacate; Caprylic/Capric Triglyceride; Polyglyceryl-3 Oleate; Diisostearoyl Polyglyceryl-3 Dimer Dilinoleate) | 4.00 |
| | Paraffin; Cera Microcristallina (Paracera W 80, Paramelt) | 0.25 |
| | Hydrogenated Castor Oil | 0.25 |
| | Tegosoft® XC MB (Phenoxyethyl Caprylate) | 7.00 |
| | Bis-Ethylhexyloxyphenol Methoxyphenyl Triazine (Tinosorb S, BASF) | 7.00 |
| | Homosalate | 10.00 |
| | Diethylamino Hydroxybenzoyl Hexyl Benzoate | 6.00 |
| | **Polyglycerol partial ester (PGE)** | **1.00** |
| | dermofeel® Toco 70 non GMO (Tocopherol, Helianthus Annuus (Sunflower) Seed Oil) | 0.50 |
| | dermosoft® GMC MB (Glyceryl Caprate) | 0.50 |
| B | Water | to 100 |
| | Glycerin | 3.00 |
| | Zinc Sulfate Heptahydrate | 1.00 |

Cationic Sun Screen SPF 19, Water Resistant

| Phase | Raw material | % w/w |
|---|---|---|
| A | Varisoft® TA 100 (Distearyldimonium Chloride) | 3.50 |
| | Tegin® M Pellets MB (Glyceryl Stearate) | 2.00 |
| | Stearyl Alcohol | 1.00 |
| | Tegosoft® CT (Caprylic/Capric Triglyceride) | 5.00 |
| | Tegosoft® DEC (Diethylhexyl Carbonate) | 3.50 |
| | **Polyglycerol partial ester (PGE)** | **1.50** |

(continued)

| Phase | Raw material | % w/w |
|---|---|---|
| | Triisostearin | 1.00 |
| | Titanium Dioxide, Diethylhexyl Carbonate, Polyglyceryl-6 Polyhyd roxystearate | 5.00 |
| | 4-Methylbenzylidene Camphor | 4.00 |
| | Octocrylene | 3.00 |
| | Butyl Methoxydibenzoylmethane | 2.00 |
| B | Glycerin | 3.00 |
| | Water | to 100 |
| Z | Preservative, Perfume | q.s. |

Cationic Sun Screen SPF 15, Water Resistant

| Phase | Raw material | % w/w |
|---|---|---|
| A | Varisoft® TA 100 (Distearyldimonium Chloride) | 3.50 |
| | Tegin® M Pellets MB (Glyceryl Stearate) | 1.50 |
| | Stearyl Alcohol | 1.00 |
| | Tegosoft® DC MB (Decyl Cocoate) | 8.00 |
| | Dicaprylyl Carbonate | 5.00 |
| | Tegosoft® MM MB (Myristyl Myristate) | 1.00 |
| | **Polyglycerol partial ester (PGE)** | **2.00** |
| | Tegosoft® OER MB (Oleyl Erucate) | 1.00 |
| | Ethylhexyl Methoxycinnamate | 5.00 |
| | Bis-Ethylhexyloxyphenol Methoxyphenyl Triazine | 3.00 |
| B | Tego® Cosmo C 100 (Creatine) | 0.50 |
| | Glycerin | 3.00 |
| | Water | to 100 |
| Z | Preservative, Perfume | q.s. |

Everyday Sunshine Cream SPF 15

| Phase | Raw material | % w/w | % w/w | % w/w |
|---|---|---|---|---|
| A | Tego® Care 165 (Glyceryl Stearate; PEG-100 Stearate) | 3.00 | 3.00 | - |
| | Polysorbate 60 | - | - | 4.00 |
| | Stearyl Alcohol | 2.00 | 1.50 | 3.00 |
| | Tegosoft® CT (Caprylic/Capric Triglyceride) | 4.00 | 4.00 | 4.00 |
| | Propylene Glycol Dibenzoate | 2.00 | 2.00 | 2.00 |
| | Titanium Dioxide; Trimethoxycaprylylsilane | 1.00 | 1.00 | 1.00 |
| | Paraffinum Liquidum | 2.00 | 2.00 | 2.00 |

(continued)

| Phase | Raw material | % w/w | % w/w | % w/w |
|---|---|---|---|---|
| | Butyrospermum Parkii (Shea Butter) | 1.00 | 1.00 | 1.00 |
| | Octocrylene | 2.00 | 2.00 | 2.00 |
| | Butyl Methoxydibenzoylmethane | 2.00 | 2.00 | 2.00 |
| | Ethylhexyl Salicylate | 5.00 | 5.00 | 5.00 |
| | **Polyglycerol partial ester (PGE)** | **1.50** | **1.50** | **1.50** |
| | Tocopheryl Acetate | 0.80 | 0.80 | 0.80 |
| B | Water | to 100 | to 100 | to 100 |
| | Glycerin | 3.00 | 3.00 | 3.00 |
| | Acrylates Copolymer | - | 1.00 | - |
| | Graham's salt (Sodium Hexametaphosphate) | 0.20 | 0.20 | 0.20 |
| | Tego® Cosmo C 100 (Creatine) | 0.50 | 0.50 | 0.50 |
| | HyaCare® 50 (Hydrolysed Hyaluronic Acid) | 0.10 | 0.10 | 0.10 |
| | Polyacrylamide; C13-14 Isoparaffin; Laureth-7 (Sepigel 305, Seppic) | 2.00 | 1.20 | 2.00 |
| C | Water | 13.29 | 13.29 | 13.29 |
| | Phenylbenzimidazole Sulfonic Acid (Eusolex 232, Merck KGaA) | 1.05 | 1.05 | 1.05 |
| | Sodium Hydroxide | q.s. | q.s. | q.s. |
| Z | Preservative, perfume | q.s. | q.s. | q.s. |

High Sun Protection Lotion O/W SPF 50

| Phase | Raw material | % w/w | % w/w | % w/w |
|---|---|---|---|---|
| A | Polyglyceryl-3 Distearate; Glyceryl Stearate Citrate | 3.50 | 3.00 | 3.00 |
| | Sodium Cetearyl Sulfate | - | 0.50 | 0.50 |
| | Tegosoft® CT (Caprylic/Capric Triglyceride) | 5.00 | 5.00 | 3.00 |
| | **Polyglycerol partial ester (PGE)** | **0.50** | **0.50** | **0.50** |
| | Rewopal® PIB 1000 (Polyisobutene) | 0.50 | 0.50 | 0.50 |
| | Acrylates/C10-30 Alkyl Acrylate Crosspolymer | 0.20 | 0.20 | - |
| | Xanthan Gum (Keltrol CG-SFT, CP Kelco) | 0.20 | 0.20 | 0.05 |
| | Titanium Dioxide; Trimethoxycaprylylsilane | 1.50 | 1.50 | 1.50 |
| | Bis-Ethylhexyloxyphenol Methoxyphenyl Triazine (Tinosorb S, BASF SE) | 4.00 | 4.00 | 4.00 |
| | Octocrylene | 10.00 | 10.00 | 10.00 |
| | Butyl Methoxydibenzoylmethane | 4.00 | 4.00 | 4.00 |
| B | Glycerin | 4.00 | to 100 | to 100 |
| | Water | to 100 | 5.00 | 5.00 |
| C | Alcohol | 5.00 | q.s. | q.s. |
| | Tegosoft® CT (Caprylic/Capric Triglyceride) | - | - | 2.20 |
| | Disteardimonium Hectorite | - | - | 0.20 |
| | Propylene Carbonate | - | - | 0.20 |

(continued)

| Phase | Raw material | % w/w | % w/w | % w/w |
|---|---|---|---|---|
| | Preservative | q.s. | q.s. | q.s. |
| D | Methylene Bis-Benzotriazolyl Tetramethylbutylphenol (Tinosorb M, BASF SE) | 8.00 | q.s. | q.s. |
| E | Sodium Hydroxide (10% in water) | q.s. to pH 6 | q.s. to pH 6 | q.s. to pH 6 |

Icy O/W Sun Care Lotion SPF 25

| Phase | Raw material | % w/w | % w/w | % w/w | % w/w |
|---|---|---|---|---|---|
| A | Polyglyceryl-3 Distearate | 3.00 | 1.00 | - | - |
| | Sucrose Polystearate; Cetyl Palmitate | - | - | 2.50 | 3.20 |
| | Sodium Stearoyl Glutamate | - | 1.50 | 1.00 | - |
| | Stearyl Alcohol | 1.00 | 1.00 | 1.00 | 1.00 |
| | dermofeel® sensolv MB (Isoamyl Laurate) | 5.00 | 5.00 | 5.00 | 5.00 |
| | Tegosoft® SH (Stearyl Heptanoate) | 1.50 | 1.50 | 1.50 | 1.50 |
| | **Polyglycerol partial ester (PGE)** | **1.50** | **1.80** | **1.80** | **1.80** |
| | Tego® Xymenynic (Caprylic/Capric Triglyceride, Xymenynic Acid) | 1.00 | 1.00 | 1.00 | 1.00 |
| | Diethylamino Hydroxybenzoyl Hexyl Benzoate | 6.00 | 6.00 | 6.00 | 6.00 |
| | 4-Methylbenzylidene Camphor | 4.00 | 4.00 | 4.00 | 4.00 |
| | Diethylhexyl Butamido Triazone | 3.00 | 3.00 | 3.00 | 3.00 |
| | Xanthan Gum (Keltrol CG-SFT, CP Kelco) | 0.50 | 0.50 | 0.50 | 0.50 |
| B | Water | to 100 | to 100 | to 100 | to 100 |
| | Glycerin | 3.00 | 3.00 | 3.00 | 3.00 |
| C | Carbomer | 0.10 | 0.10 | 0.10 | 0.10 |
| | Tegosoft® XC MB (Phenoxyethyl Caprylate) | 0.40 | 0.40 | 0.40 | 0.40 |
| D | Sodium Hydroxide (10% in water) | q.s. | q.s. | q.s. | q.s. |
| E | Preservative | q.s. | q.s. | q.s. | q.s. |
| Z | Perfume | q.s. | q.s. | q.s. | q.s. |

Low viscosity W/O sun care lotion SPF30 PA+++

| Phase | Raw material | % w/w | % w/w |
|---|---|---|---|
| A | Abil® EM 90 (Cetyl PEG/PPG-10/1 Dimethicone) | 2.50 | 2.50 |
| | Tegosoft® TN (C12-15 Alkyl Benzoate) | 2.00 | 3.00 |
| | Dicaprylyl Carbonate | 2.00 | 2.00 |
| | Tegosoft® CT (Caprylic/Capric Triglyceride) | 1.00 | - |
| | **Polyglycerol partial ester (PGE)** | **0.40** | **1.60** |
| | Dimethicone (DC 200, 5 cSt, Dow Corning) | 16.00 | 16.00 |
| | Titanium Dioxide (Parsol TX, DSM) | 10.00 | 10.00 |

(continued)

| Phase | Raw material | % w/w | % w/w |
|---|---|---|---|
| | Zinc Oxide (Sunclear-Z60CO, Sunjin) | 25.00 | 25.00 |
| | Hydrogenated Lecithin | 0.50 | 1.50 |
| | Dimethicone/Vinyl Dimethicone Crosspolymer and Silica (DC 9701, DowCorning) | 1.00 | 1.00 |
| | Nylon-12 | 1.50 | 1.50 |
| B | Water | to 100 | to 100 |
| | Butylene Glycol | 5.00 | 5.00 |
| | Sodium Chloride | 1.50 | 1.50 |
| C | Phenoxyethanol, Ethylhexylglycerin (Euxyl PE 9010, Schülke & Mayr GmbH) | 0.70 | 0.70 |

Sun Care Aqua Gel SPF50+,PA++++

| Phase | Raw material | % w/w |
|---|---|---|
| A | Abil® Care XL 80 (Bis-PEG/PPG-20/5 PEG/PPG-20/5 Dimethicone; Methoxy PEG/PPG-25/4 Dimethicone; Caprylic/Capric Triglyceride) | 3.00 |
| | Tegosoft® DEC (Diethylhexyl Carbonate) | 7.00 |
| | Dimethicone (5 cSt) | 2.00 |
| | Tegosoft® CT (Caprylic/Capric Triglyceride) | 3.00 |
| | Abil® Wax 9801 (Cetyl Dimethicone) | 2.00 |
| | Bis-ethylhexyloxyphenol Methoxyphenyl Triazine (Tinosorb S, BASF) | 3.00 |
| | Diethylamino Hydroxybenzoyl Hexyl Benzoate (Uvinul A plus Granular, BASF) | 4.00 |
| | Octocrylene | 5.00 |
| | Avobenzone (Parsol 1789, DSM) | 3.00 |
| | Homomenthyl Salicylate | 8.00 |
| | Ethylhexyl Salicylate | 5.00 |
| | Ethylhexyl Methoxycinnamate | 10.00 |
| | **Polyglycerol partial ester (PGE)** | 1.50 |
| B | Water | to 100 |
| | Butylene Glycol | 3.00 |
| | Xanthan Gum (Keltrol CG-SFT, CP Kelco) | 0.20 |
| C | Sodium Acrylate/Sodium Acryloyldimethyl Taurate Copolymer (and) Isohexadecane (and) Polysorbate-80 (Simulgel EG, SEPPIC) | 1.50 |
| D | Phenoxyethanol, Ethylhexylglycerin (Euxyl PE 9010, Schülke & Mayr GmbH) | 0.70 |

O/W Sun Care Cream with high protection SPF 50+ PA+++

| Phase | Raw material | % w/w | % w/w | % w/w | % w/w |
|---|---|---|---|---|---|
| A | Tego® Care 450 MB (Polyglyceryl-3 Methylglucose Distearate) | 3.00 | 3.00 | 2.50 | 2.50 |
| | Potassium Cetyl Phosphate | - | 0.50 | - | - |

(continued)

| Phase | Raw material | % w/w | % w/w | % w/w | % w/w |
|---|---|---|---|---|---|
| | Glyceryl Stearate Citrate | - | - | 0.60 | - |
| | Sodium Stearoyl Glutamate | - | - | - | 0.70 |
| | Caprylic/Capric Triglyceride | 2.00 | 2.00 | - | - |
| | Butylene Glycol Dicaprylate/Dicaprate | - | - | 1.50 | 2.00 |
| | Tego® Alkanol 1618 (Cetearyl Alcohol) | 1.00 | 1.00 | 1.00 | 1.00 |
| | Abil® Wax 9840 (Cetyl Dimethicone) | 0.50 | 0.50 | 0.50 | 0.50 |
| | Bis-ethylhexyloxyphenol Methoxyphenyl Triazine (Tinosorb S, BASF SE) | 2.00 | 2.00 | 2.00 | 2.00 |
| | Butyl Methoxydibenzoylmethane | 3.00 | 3.00 | 3.00 | 3.00 |
| | Octocrylene | 8.00 | 8.00 | 8.00 | 8.00 |
| | Ethylhexyl Salicylate | 5.00 | 5.00 | 5.00 | 5.00 |
| | **Polyglycerol partial ester (PGE)** | **2.60** | **1.90** | **2.80** | **2.30** |
| | C12-15 Alkyl Benzoate; Titanium Dioxide; PEG-30 Dipolyhydroxystearate; Silica; Dimethicone (T-lique AB50Si, Uni-Powder) | 8.90 | 8.90 | 8.90 | 8.90 |
| B | Water | to 100 | to 100 | to 100 | to 100 |
| | Glycerin | 3.00 | 3.00 | 3.00 | 3.00 |
| C | Phenylbenzimidazole Sulfonic Acid | 3.00 | 3.00 | 3.00 | 3.00 |
| | Tromethamine 78% | 2.00 | 2.00 | 2.00 | 2.00 |
| | Water | 4.00 | 4.00 | 4.00 | 4.00 |
| D | Acrylates/C10-30 Alkyl Acrylate Crosspolymer (2% solution in water) | 10.00 | 10.00 | 10.00 | 10.00 |
| E | Sodium Hydroxide (10% in water) | 0.40 | 0.40 | 0.40 | 0.40 |
| F | Phenoxyethanol, Ethylhexylglycerin (Euxyl PE 9010, Schülke & Mayr GmbH) | 0.70 | 0.70 | 0.70 | 0.70 |

Sun Care Cream SPF 15

| Phase | Raw material | % w/w | % w/w | % w/w | % w/w | % w/w |
|---|---|---|---|---|---|---|
| A | Tego® Care 450 MB (Polyglyceryl-3 Methylglucose Distearate) | 3.00 | 2.50 | 2.50 | 2.50 | 2.50 |
| | Potassium Cetyl Phosphate | - | 0.80 | 0.80 | 0.80 | 0.80 |
| | Tegin® M Pellets MB (Glyceryl Stearate) | 1.50 | 1.50 | 1.50 | 1.50 | 1.50 |
| | Tego® Alkanol 1618 (Cetearyl Alcohol) | 1.50 | 1.50 | 1.50 | 1.50 | 1.50 |
| | Tegosoft® CT (Caprylic/Capric Triglyceride) | 2.00 | - | - | - | - |
| | Dibutyl Adipate | - | 5.00 | 5.00 | 5.00 | 5.00 |
| | dermofeel® sensolv MB (Isoamyl Laurate) | 7.00 | 5.00 | 5.00 | 5.00 | 5.00 |
| | Tegosoft® CR MB (Cetyl Ricinoleate) | 1.00 | 1.00 | 1.00 | 1.00 | 1.00 |
| | **Polyglycerol partial ester (PGE)** | **3.00** | **1.00** | **1.30** | **1.40** | **1.40** |
| | Capryloyl Glycerin/Sebacic Acid Copolymer (LexFilm Sun Natural, Inolex) | - | 1.20 | - | - | - |

(continued)

| Phase | Raw material | % w/w | % w/w | % w/w | % w/w | % w/w |
|---|---|---|---|---|---|---|
| | Polyglyceryl-3 Stearate/Isostearate/Dimer Dilinoleate Crosspolymer (CosmoSurf PG1-IS, SurfaTech Corp.) | - | - | 1.00 | - | - |
| | Diisostearoyl Polyglyceryl-3 Dimer Dilinoleate (and) Caprylic/Capric Triglyceride (SolAmaze Natural polymer, Nouryon) | - | - | - | 0.80 | - |
| | Sorbitol/Sebacic Acid Copolymer Behenate (Syncrowax ORM, Croda) | - | - | - | - | 1.00 |
| | Bis-Ethylhexyloxyphenol Methoxyphenyl Triazine (Tinosorb S, BASF) | 1.50 | 1.50 | 1.50 | 1.50 | 1.50 |
| | Butyl Methoxydibenzoylmethane | 1.00 | 1.00 | 1.00 | 1.00 | 1.00 |
| | Ethylhexyl Triazone | 0.75 | 0.75 | 0.75 | 0.75 | 0.75 |
| | Homomenthyl Salicylate | 1.50 | 1.50 | 1.50 | 1.50 | 1.50 |
| | Ethylhexyl Salicylate | 1.50 | 1.50 | 1.50 | 1.50 | 1.50 |
| | Octocrylene | 4.00 | 4.00 | 4.00 | 4.00 | 4.00 |
| | dermofeel® TocoBalance (Tocopherol, Helianthus Annuus (Sunflower) Seed Oil) | 0.70 | 0.70 | 0.70 | 0.70 | 0.70 |
| | Tego® Feel C 10 (Cellulose) | 2.00 | 2.00 | 2.00 | 2.00 | 2.00 |
| | Tego® SP 13-1 (Poly C10-30 Alkyl Acrylate) | 1.50 | - | - | - | - |
| | Phytosphingosine SLC (Salicyloyl Phytosphingosine) | 0.10 | 0.10 | 0.10 | 0.10 | 0.10 |
| B | Water | to 100 | to 100 | to 100 | to 100 | to 100 |
| | Glycerin | 3.00 | 3.00 | 3.00 | 3.00 | 3.00 |
| | EDTA | 0.05 | 0.05 | 0.05 | 0.05 | 0.05 |
| | Tego® Cosmo C 100 (Creatine) | 0.50 | 0.50 | 0.50 | 0.50 | 0.50 |
| C | Xanthan Gum (Ketrol CG-SFT, CP Kelco) | 0.50 | 0.50 | 0.50 | 0.50 | 0.50 |
| D | Tego® Pep Up (Tetrapeptide-4; Glycerin) | 2.00 | 2.00 | 2.00 | 2.00 | 2.00 |
| E | Verstatil® PC (Phenoxyethanol, Caprylyl Glycol) | 1.20 | 1.20 | 1.20 | 1.20 | 1.20 |
| | Perfume | q.s. | q.s. | q.s. | q.s. | q.s. |
| | Tromethamine (Trisaminomethane, 30% in water) | q.s. | q.s. | q.s. | q.s. | q.s. |

W/O foundation

| Phase | Raw material | % w/w | % w/w |
|---|---|---|---|
| A | Isolan® GPS (Polyglyceryl-4 Diisostearate / Polyhydroxystearate / Sebacate) | 4.00 | - |
| | Polyglyceryl-6 Polyhydroxystearate; Polyglyceryl-6 Polyricinoleate (Emulium Illustro, Gattefossé) | - | 3.80 |
| | Tegosoft® AC MB (Isoamyl Cocoate) | 5.50 | 5.50 |
| | Tegosoft® DC MB (Decyl Cocoate) | 7.50 | 7.50 |
| | Tegosoft® CT (Caprylic/Capric Triglyceride) | 3.50 | 3.50 |
| | CI 77891, Hydrogenated Lecithin (Unipure White LC 981 HLC, Sensient) | 15.00 | 15.00 |

(continued)

| Phase | Raw material | % w/w | % w/w |
|---|---|---|---|
|  | CI 77492, Hydrogenated Lecithin (Unipure Yellow LC 182 HLC, Sensient) | 1.20 | 1.20 |
|  | CI 77491, Hydrogenated Lecithin (Unipure Red LC 381 HLC Sensient) | 0.70 | 0.70 |
|  | CI 77499, Hydrogenated Lecithin (Unipure Black LC 989 HLC, Sensient) | 0.20 | 0.20 |
|  | **Polyglycerol partial ester (PGE)** | 1.50 | 1.50 |
| B | Water | to 100 | to 100 |
|  | Glycerin | 3.00 | 3.00 |
|  | Zinc Sulfate Heptahydrate | 2.00 | 2.00 |
|  | Verstatil® SL non GMO (Sodium Levulinate, Potassium Sorbate, Water) | q.s. | q.s. |
|  | Citric Acid (10% in water) | q.s. to pH 4.4 | q.s. to pH 4.4 |

W/O foundation

| Phase | Raw material | % w/w |
|---|---|---|
| A | Isolan® 17 MB (Polyglyceryl-4 Diisostearate/ Polyhydroxystearate/ Sebacate; Caprylic/Capric Triglyceride; Polyglyceryl-3 Oleate; Diisostearoyl Polyglyceryl-3 Dimer Dilinoleate) | 4.00 |
|  | Isododecane | 3.33 |
|  | Tegosoft® XC MB (Phenoxyethyl Caprylate) | 3.33 |
|  | Dimethicone | 3.33 |
|  | **Polyglycerol partial ester (PGE)** | **2.00** |
|  | CI 77891, Titanium Dioxide, Alumina, Triethoxycaprylylsilane (Hombitan AC360, Sachtleben) | 4.00 |
|  | CI 77491, CI 77492, CI 77499 | 2.50 |
|  | (Iron Oxide, Sicovit Brown 70 E 172, Rockwood Pigments) |  |
|  | Talc | 2.00 |
| B | Dicaprylyl Carbonate, Stearalkonium Hectorite, Propylene Carbonate (Cosmedia Gel CC, BASF SE) | 3.00 |
|  | Isododecane | 1.50 |
|  | Tegosoft® XC MB (Phenoxyethyl Caprylate) | 1.50 |
|  | Dimethicone | 1.50 |
| C | Glycerin | 5.00 |
|  | Magnesium Sulfate Heptahydrate | 1.50 |
|  | Water | to 100 |
| D | Verstatil® PC (Phenoxyethanol, Caprylyl Glycol) | 1.00 |
|  | Boron Nitride (Boroneige 1201, ESK) | 5.00 |

SPF 30 sun lotion with water-soluble emulsifier and natural, aqueous thickeners

EP 4 279 531 B1

| Phase | Raw material | % w/w | % w/w | % w/w | % w/w | % w/w | % w/w | % w/w | % w/w |
|---|---|---|---|---|---|---|---|---|---|
| A | Tegin® M Pellets MB (Glyceryl Stearate) | 0.5 | 0.5 | 0.5 | 0.5 | 0.5 | 0.5 | 0.5 | 0.5 |
| | Tego® Alkanol 1618 (Cetearyl Alcohol) | 0.5 | 0.5 | 0.5 | 0.5 | 0.5 | 0.5 | 0.5 | 0.5 |
| | Tegosoft® XC MB (Phenoxyethyl Caprylate) | 4.3 | 4.3 | 4.3 | 4.3 | 4.3 | 4.3 | 4.3 | 4.3 |
| | Polyglycerol partial ester (PGE) | 1.5 | 1.5 | 1.5 | 1.5 | 1.5 | 1.5 | 1.5 | 1.5 |
| | Bis-ethylhexyloxyphenol Methoxyphenyl Triazine | 3 | 3 | 3 | 3 | 3 | 3 | 3 | 3 |
| | Butyl Methoxy Dibenzoylmethane | 2 | 2 | 2 | 2 | 2 | 2 | 2 | 2 |
| | Ethylhexyl Salicylate | 3 | 3 | 3 | 3 | 3 | 3 | 3 | 3 |
| | Octocrylene | 8 | 8 | 8 | 8 | 8 | 8 | 8 | 8 |
| | Homosalate | 3 | 3 | 3 | 3 | 3 | 3 | 3 | 3 |
| | Ethylhexyl Triazone | 1.5 | 1.5 | 1.5 | 1.5 | 1.5 | 1.5 | 1.5 | 1.5 |
| | dermofeel® Toco 70 non-GMO (Tocopherol, Helianthus Annuus (Sunflower) Seed Oil) | 0.2 | 0.2 | 0.2 | 0.2 | 0.2 | 0.2 | 0.2 | 0.2 |
| B | Potassium Cetyl Phosphate | 1.5 | 1.5 | 1.5 | 1.5 | 1.5 | 1.5 | 1.5 | 1.5 |
| | Glycerin | 3 | 3 | 3 | 3 | 3 | 3 | 3 | 3 |
| | Water | to 100 | to 100 | to 100 | to 100 | to 100 | to 100 | to 100 | to 100 |
| | EDTA | 0.05 | 0.05 | 0.05 | 0.05 | 0.05 | 0.05 | 0.05 | 0.05 |
| C | Xanthan Gum (Keltrol CG-SFT, CP Kelco) | 0.3 | | | | | | | |
| | Succinoglycan (Rheozan® SH, Solvay) | | 0.3 | | | | | | |
| | Glucomannan (Rheolex® One-C, Nagase) | | | 0.3 | | | | | |
| | Sclerotium Gum (Amigel®, Alban Muller) | | | | 0.4 | | | | |
| | Gellan Gum (Kelcogel® Gellan Gum, CP Kelco) | | | | | 0.1 | | | |
| | Caesalpinia Spinosa Gum (Solagum® Tara, Seppic) | | | | | | 0.45 | | |
| | Cellulose (Exilva® 10 w-% active matter, Borregaard) | | | | | | | 1 | |
| D | Phenoxyethanol, Caprylyl Glycol | 1 | 1 | 1 | 1 | 1 | 1 | 1 | 1 |

SPF 30 sun lotion with oil-soluble emulsifiers and natural, aqueous thickeners

| Phase | Raw material | % w/w | % w/w | % w/w | % w/w | % w/w | % w/w | % w/w | % w/w | % w/w | % w/w | % w/w |
|---|---|---|---|---|---|---|---|---|---|---|---|---|
| A | Tego® Care PBS 6 MB (Polyglyceryl-6 Stearate, Polyglyceryl-6 Behenate) | 3 | 3 | 3 | | | | | | 3 | | |

44

(continued)

| Phase | Raw material | % w/w | % w/w | % w/w | % w/w | % w/w | % w/w | % w/w | % w/w | % w/w | % w/w | % w/w | % w/w |
|---|---|---|---|---|---|---|---|---|---|---|---|---|---|
| | Axol® C 62 Pellets MB (Glyceryl Stearate Citrate) | | | 3 | 3 | 3 | | | | | | 3 | |
| | Tego® Care 450 MB (Polyglyceryl-3 Methylglucose Distearate) | | | | | | 3 | 3 | 3 | | | | 3 |
| | Tegin® M Pellets MB (Glyceryl Stearate) | 0.5 | 0.5 | 0.5 | 0.5 | 0.5 | 0.5 | 0.5 | 0.5 | 0.5 | 0.5 | 0.5 | 0.5 |
| | Tego® Alkanol 1618 (Cetearyl Alcohol) | 0.5 | 0.5 | 0.5 | 0.5 | 0.5 | 0.5 | 0.5 | 0.5 | 0.5 | | 0.5 | 0.5 |
| | Tegosoft® XC MB (Phenoxyethyl Caprylate) | 4.3 | 4.3 | 4.3 | 4.3 | 4.3 | 4.3 | 4.3 | 4.3 | 4.3 | 4.3 | 4.3 | 4.3 |
| | **Polyglycerol partial ester (PGE)** | **1.5** | **1.5** | **1.5** | **1.5** | **1.5** | **1.5** | **1.5** | **1.5** | **1.5** | **3.0** | **3.0** | **3.0** |
| | Bis-ethylhexyloxyphenol Methoxyphenyl Triazine | 3 | 3 | 3 | 3 | 3 | 3 | 3 | 3 | 3 | 3 | 3 | 3 |
| | Butyl Methoxy Dibenzoylmethane | 2 | 2 | 2 | 2 | 2 | 2 | 2 | 2 | 2 | 2 | 2 | 2 |
| | Ethylhexyl Salicylate | 3 | 3 | 3 | 3 | 3 | 3 | 3 | 3 | 3 | 3 | 3 | 3 |
| | Octrocrylene | 8 | 8 | 8 | 8 | 8 | 8 | 8 | 8 | 8 | 8 | 8 | 8 |
| | Homosalate | 3 | 3 | 3 | 3 | 3 | 3 | 3 | 3 | 3 | 3 | 3 | 3 |
| | Ethylhexyl Triazone | 1.5 | 1.5 | 1.5 | 1.5 | 1.5 | 1.5 | 1.5 | 1.5 | 1.5 | 1.5 | 1.5 | 1.5 |
| | dermofeel® Toco 70 non-GMO (Tocopherol, Helianthus Annuus (Sunflower) Seed Oil) | 0.2 | 0.2 | 0.2 | 0.2 | 0.2 | 0.2 | 0.2 | 0.2 | 0.2 | 0.2 | 0.2 | 0.2 |
| B | Glycerin | 3 | 3 | 3 | 3 | 3 | 3 | 3 | 3 | 3 | 3 | 3 | 3 |
| | Water | to 100 | to 100 | to 100 | to 100 | to 100 | to 100 | to 100 | to 100 | to 100 | to 100 | to 100 | to 100 |
| | EDTA | 0.05 | 0.05 | 0.05 | 0.05 | 0.05 | 0.05 | 0.05 | 0.05 | 0.05 | 0.05 | 0.05 | 0.05 |
| C | Xanthan Gum (Keltrol CG-SFT, CP Kelco) | 0.3 | | | 0.3 | | | 0.3 | | | 0.3 | 0.3 | 0.3 |
| | Succinoglycan (Rheozan® SH, Solvay) | | 0.3 | | | 0.3 | | | 0.3 | | | | |
| | Glucomannan (Rheolex® One-C, Nagase) | | | 0.3 | | | 0.3 | | | 0.3 | | | |
| D | Phenoxyethanol, Caprylyl Glycol | 1 | 1 | 1 | 1 | 1 | 1 | 1 | 1 | 1 | 1 | 1 | 1 |

SPF 30 sun lotion, mineral UF filters and natural, aqueous thickener

| Phase | Raw material | % w/w | % w/w | % w/w | % w/w |
|---|---|---|---|---|---|
| A 1 | Tego® Care PBS 6 MB (Polyglyceryl-6 Stearate, Polyglyceryl-6 Behenate) | 4 | | | |
| | Axol® C 62 Pellets MB (Glyceryl Stearate Citrate) | | 2.5 | | |
| | Tego® Care 450 MB (Polyglyceryl-3 Methylglucose Distearate) | | | 3 | |
| | Tego® Alkanol 1618 (Cetearyl Alcohol) | 0.2 | 0.2 | 0.2 | 0.2 |
| | Tegin® M Pellets MB (Glyceryl Stearate) | 0.2 | 0.2 | 0.2 | 0.2 |
| | **Polyglycerol partial ester (PGE)** | **1.5** | **1.5** | **1.5** | **1.5** |
| A 2 | Tegosoft® CT (Caprylic/Capric Triglyceride) | 5 | 5 | 5 | 5 |
| | dermofeel® sensolv MB (Isoamyl Laurate) | 6 | 6 | 6 | 6 |
| | Tegosoft® XC (Phenoxyethyl Caprylate) | | | | |
| | Tegosoft® DEC (Diethylhexyl Carbonate) | 5 | 5 | 5 | 5 |
| | Dimethicone | 3 | 3 | 3 | 3 |
| | Abil® Wax 9840 (Cetyl Dimethicone) | 2 | 2 | 2 | 2 |
| | Titanium Dioxide, Silica; Dimethicone (Parsol® TX, DSM) | 7.2 | 7.2 | 7.2 | 7.2 |
| | Zinc Oxide, Triethoxycaprylylsilane (Z-Cote® HP 1, BASF) | 7.8 | 7.8 | 7.8 | 7.8 |
| | dermosoft® Octiol (Caprylyl Glycol) | 0.5 | 0.5 | 0.5 | 0.5 |
| B | Potassium Cetyl Phosphate | | | | 1.5 |
| | Water | to 100 | to 100 | to 100 | to 100 |
| | Glycerin | 3 | 3 | 3 | 3 |
| | Xanthan Gum (Keltrol CG-SFT, CP Kelco) | 0.5 | 0.5 | 0.5 | 0.5 |
| D | dermosoft® Pentiol eco (Pentylene Glycol.) | 3 | 3 | 3 | 3 |
| E | Citric Acid 50% w-% in water | 0.45 | 0.45 | 0.45 | 0.45 |

**Claims**

1.  Polyglycerol partial ester obtainable by esterification

    a) of a polyglycerol having an average degree of condensation N of 2.4 to 15.0, preferably 2.6 to 10.0, especially 2.8 to 6.0,

    with a carboxylic acid mixture comprising:

    b) at least one short-chain dicarboxylic acid having 4 to 18, preferably 4 to 14, more preferably 6 to 10, carbon atoms and
    c) at least one long-chain, saturated and linear monocarboxylic acid having 18 to 32, preferably 18 to 26, more preferably 18 to 22, carbon atoms,
    wherein the sum total of all short-chain dicarboxylic acids present in the carboxylic acid mixture comes to at least 75% by weight, preferably at least 85% by weight, more preferably at least 95% by weight, based on all the dicarboxylic acids present in the carboxylic acid mixture,
    wherein the sum total of all long-chain, saturated and linear monocarboxylic acids present in the carboxylic acid mixture comes to at least 86% by weight, preferably at least 90% by weight, more preferably at least 95% by weight, based on all the monocarboxylic acids present in the carboxylic acid mixture,
wherein the molar ratio of all carboxyl groups present in the carboxylic acid mixture to all hydroxyl groups present in the polyglycerol used is at least 50 to 100, preferably at least 55 to 98, more preferably at least 68 to 95,
**characterized in that**
the molar ratio of polyglycerol a) to dicarboxylic acid component b) to monocarboxylic acid component c) is from 1.0:1.0:2.1 to 4.0:1.0:10.0, preferably from 1.0:1.0:3.1 to 3.0:1.0:8.0.

2. Polyglycerol partial ester according to Claim 1, **characterized in that** the polyglycerol used has a glycerin content of 0.05% by weight to 25.0% by weight, preferably of 0.1% by weight to 15.0% by weight, more preferably of 0.1% by weight to 10.0% by weight.

3. Polyglycerol partial ester according to Claim 1 or 2, **characterized in that** the polyglycerol used has a diglycerin content of 0.1% by weight to 45.0% by weight, preferably of 0.5% by weight to 35.0% by weight, more preferably of 3.0% by weight to 30.0% by weight.

4. Polyglycerol partial ester according to at least one of the preceding claims, **characterized in that** the polyglycerol used has a content of cyclic isomers of 1% by weight to 50% by weight, preferably of 2% by weight to 40% by weight, more preferably of 3% by weight to 30% by weight.

5. Polyglycerol partial ester according to at least one of the preceding claims, **characterized in that** the short-chain dicarboxylic acid is selected from aliphatic, linear dicarboxylic acids, especially oxalic acid, malonic acid, tartronic acid, succinic acid, maleic acid, tartaric acid, malic acid, fumaric acid, sorbic acid, $\alpha$-ketoglutaric acid, glutaric acid, adipic acid, pimelic acid, suberic acid, azelaic acid, sebacic acid, undecanedioic acid, dodecanedioic acid and brassylic acid, with particular preference given to adipic acid, pimelic acid, suberic acid, azelaic acid and sebacic acid.

6. Polyglycerol partial ester according to at least one of the preceding claims, **characterized in that** the long-chain, saturated and linear monocarboxylic acids are selected from hydroxy-substituted and unsubstituted, especially unsubstituted, fatty acids.

7. Polyglycerol partial ester according to at least one of the preceding claims, **characterized in that** the total content of aromatic mono- and dicarboxylic acids based on all mono- and dicarboxylic acids used is from 0.1% by weight to 35% by weight, preferably from 0% by weight to 25% by weight, more preferably from 0% by weight to 10% by weight.

8. Polyglycerol partial ester according to at least one of the preceding claims, **characterized in that** it has a degree of esterification of 50% to 100%, preferably of 55% to 98%, more preferably of 68% to 95%.

9. Polyglycerol partial ester according to at least one of the preceding claims, **characterized in that** it has a HLB value of from 2.0 to 8.0, preferably from 2.5 to 6 and more preferably from 3 to 5.2, measured as defined in the specification.

10. Polyglycerol partial ester according to at least one of the preceding claims, **characterized in that** it has an iodine value of less than 20, preferably less than 12, particularly preferably less than 5, measured as defined in the specification.

11. Polyglycerol partial ester according to at least one of the preceding claims, **characterized in that** it has a melting point of greater than 35°C, preferably greater than 40°C, in particular greater than 50°C, more preferably within a range from 51°C to 100°C, measured as defined in the specification.

12. Method for preparing a polyglycerol partial ester, comprising the method steps of

A) providing a polyglycerol having an average degree of condensation N of 2.4 to 15.0, preferably 2.6 to 10.0, especially 2.8 to 6.0,
B) providing a carboxylic acid mixture comprising:

at least one short-chain dicarboxylic acid having 4 to 18, preferably 4 to 14, more preferably 6 to 10, carbon atoms and
at least one long-chain, saturated and linear monocarboxylic acid having 18 to 32, preferably 18 to 26, more preferably 18 to 22, carbon atoms,
wherein the sum total of all short-chain dicarboxylic acids present in the carboxylic acid mixture comes to at least 75% by weight, preferably at least 85% by weight, more preferably at least 95% by weight, based on all the dicarboxylic acids present in the carboxylic acid mixture,

wherein the sum total of all long-chain, saturated and linear monocarboxylic acids present in the carboxylic acid mixture comes to at least 75% by weight, preferably at least 85% by weight, more preferably at least 95% by weight, based on all the monocarboxylic acids present in the carboxylic acid mixture,

C) esterifying the polyglycerol with the carboxylic acid mixture,
wherein the molar ratio of all carboxyl groups present in the carboxylic acid mixture to all hydroxyl groups present in the polyglycerol used is at least 50 to 100, preferably at least 55 to 98, more preferably at least 68 to 95,
**characterized in that** the molar ratio of polyglycerol a) to dicarboxylic acid component b) to carboxylic acid component c) is from 1.0:1.0:2.1 to 4.0:1.0:10.0, preferably from 1.0:1.0:3.1 to 3.0:1.0:8.0.

13. Formulation, especially a cosmetic formulation, comprising

1) polyglycerol partial esters according to at least one of Claims 1 to 11 or obtainable according to the method of Claim 12, and optionally
2) at least one substance selected from the group comprising UV light protection filter substances and pigments, especially from the group of UV light protection filter substances.

14. Formulation according to Claim 13, **characterized in that** component 2) is selected from the group of UV light protection filter substances comprising butyl methoxydibenzoylmethane, ethylhexyl triazone, bis-ethylhexyloxyphenol methoxyphenyl triazine, diethylhexyl butamido triazone, diethylamino hydroxybenzoyl hexyl benzoate, benzophenone-3, benzophenone-4, 4-methylbenzylidene camphor, octocrylene, ethylhexyl methoxycinnamate, ethylhexyl salicylate, homomenthyl salicylate, phenylbenzimidazole sulfonic acid, methylene bis-benzotriazolyl tetramethylbutylphenol, disodium phenyl dibenzimidazole tetrasulfonate, isoamyl p-methoxycinnamate and ethylhexyl dimethyl PABA and/or
is selected from the group of inorganic pigments, comprising in particular activated carbon, talc, iron oxide pigments, titanium dioxide, zinc oxide, silica, cerium oxides, zirconium oxides, aluminium oxides, calcium carbonate, barium sulfate, chromium oxides, manganese oxides, bismuth oxychloride, ultramarine, calcium sulfate and alkali magnesium silicates.

15. Formulation according to Claim 13 or 14, **characterized in that**

component 1) is present in an amount of 0.1% by weight to 20% by weight, preferably of 0.25% by weight to 12% by weight, more preferably of 0.5% by weight to 6% by weight, and
component 2) is present in an amount of 0.1% by weight to 60% by weight, preferably of 1% by weight to 50% by weight, more preferably of 3% by weight to 40% by weight.

16. Use of a polyglycerol partial ester according to at least one of Claims 1 to 11 or obtainable according to the method of Claim 12 as a film former, to boost the sun protection factor of UV light protection filter substances or to reduce the rinseability and/or the wear of a formulation from a surface.

## Patentansprüche

1. Polyglycerinpartialester erhältlich durch Veresterung

a) eines Polyglycerins mit einem mittleren Kondensationsgrad N von 2,4 bis 15,0, bevorzugt 2,6 bis 10,0, insbesondere 2,8 bis 6,0,

mit einer Carbonsäuremischung umfassend:

b) mindestens eine kurzkettige Dicarbonsäure mit 4 bis 18, bevorzugt 4 bis 14, besonders bevorzugt 6 bis 10, Kohlenstoffatomen und
c) mindestens eine langkettige, gesättigte und lineare Monocarbonsäure mit 18 bis 32, bevorzugt 18 bis 26, besonders bevorzugt 18 bis 22, Kohlenstoffatomen,
wobei die Summe aller in der Carbonsäuremischung enthaltenen kurzkettigen Dicarbonsäuren mindestens 75 Gew.-%, bevorzugt mindestens 85 Gew.-%, besonders bevorzugt mindestens 95 Gew.-%, bezogen auf alle in der Carbonsäuremischung enthaltenen Dicarbonsäuren, ausmacht,

wobei die Summe aller in der Carbonsäuremischung enthaltenen langkettigen, gesättigten und linearen Monocarbonsäuren mindestens 86 Gew.-%, bevorzugt mindestens 90 Gew.-%, besonders bevorzugt mindestens 95 Gew.-%, bezogen auf alle in der Carbonsäuremischung enthaltenen Monocarbonsäuren, ausmacht,

wobei das Molverhältnis aller in der Carbonsäuremischung enthaltenen Carboxylgruppen zu allen im eingesetzten Polyglycerin enthaltenen Hydroxylgruppen mindestens 50 zu 100, bevorzugt mindestens 55 zu 98, besonders bevorzugt mindestens 68 zu 95, beträgt,

**dadurch gekennzeichnet, dass**

das Molverhältnis von Polyglycerin a) zu Dicarbonsäurekomponente b) zu Monocarbonsäurekomponente c) 1,0:1,0:2,1 bis 4,0:1,0:10,0, bevorzugt 1,0:1,0:3,1 bis 3,0:1,0:8,0, beträgt.

2. Polyglycerinpartialester nach Anspruch 1, **dadurch gekennzeichnet, dass** das eingesetzte Polyglycerin einen Glyceringehalt von 0,05 Gew.-% bis 25,0 Gew.-%, bevorzugt von 0,1 Gew.-% bis 15,0 Gew.-%, besonders bevorzugt von 0,1 Gew.-% bis 10,0 Gew.-%, aufweist.

3. Polyglycerinpartialester nach Anspruch 1 oder 2, **dadurch gekennzeichnet, dass** das eingesetzte Polyglycerin einen Diglyceringehalt von 0,1 Gew.-% bis 45,0 Gew.-%, bevorzugt von 0,5 Gew.-% bis 35,0 Gew.-%, besonders bevorzugt von 3,0 Gew.-% bis 30,0 Gew.-%, aufweist.

4. Polyglycerinpartialester nach mindestens einem der vorhergehenden Ansprüche, **dadurch gekennzeichnet, dass** das eingesetzte Polyglycerin einen Gehalt an cyclischen Isomeren von 1 Gew.-% bis 50 Gew.-%, bevorzugt von 2 Gew.-% bis 40 Gew.-%, besonders bevorzugt von 3 Gew.-% bis 30 Gew.-%, aufweist.

5. Polyglycerinpartialester nach mindestens einem der vorhergehenden Ansprüche, **dadurch gekennzeichnet, dass** die kurzkettige Dicarbonsäure ausgewählt ist aus aliphatischen, linearen Dicarbonsäuren, insbesondere Oxalsäure, Malonsäure, Tartronsäure, Bernsteinsäure, Maleinsäure, Weinsäure, Äpfelsäure, Fumarsäure, Sorbinsäure, $\alpha$-Ketoglutarsäure, Glutarsäure, Adipinsäure, Pimelinsäure, Korksäure, Azelainsäure, Sebacinsäure, Undecandisäure, Dodecandisäure und Brassylsäure, wobei Adipinsäure, Pimelinsäure, Korksäure, Azelainsäure und Sebacinsäure besonders bevorzugt sind.

6. Polyglycerinpartialester nach mindestens einem der vorhergehenden Ansprüche, **dadurch gekennzeichnet, dass** die langkettigen, gesättigten und linearen Monocarbonsäuren ausgewählt sind aus hydroxysubstituierten und unsubstituierten, insbesondere unsubstituierten, Fettsäuren.

7. Polyglycerinpartialester nach mindestens einem der vorhergehenden Ansprüche, **dadurch gekennzeichnet, dass** der Gesamtgehalt an aromatischen Mono- und Dicarbonsäuren, bezogen auf alle eingesetzten Mono- und Dicarbonsäuren, 0,1 Gew.-% bis 35 Gew.-%, bevorzugt 0 Gew.-% bis 25 Gew.-%, besonders bevorzugt 0 Gew.-% bis 10 Gew.-%, beträgt.

8. Polyglycerinpartialester nach mindestens einem der vorhergehenden Ansprüche, **dadurch gekennzeichnet, dass** er einen Veresterungsgrad von 50 % bis 100%, bevorzugt von 55 % bis 98%, besonders bevorzugt von 68 % bis 95 %, aufweist.

9. Polyglycerinpartialester nach mindestens einem der vorhergehenden Ansprüche, **dadurch gekennzeichnet, dass** er einen HLB-Wert von 2,0 bis 8,0, bevorzugt von 2,5 bis 6 und besonders bevorzugt von 3 bis 5,2, gemessen wie in der Beschreibung definiert, aufweist.

10. Polyglycerinpartialester nach mindestens einem der vorhergehenden Ansprüche, **dadurch gekennzeichnet, dass** er eine Iodzahl von kleiner 20, bevorzugt kleiner 12, besonders bevorzugt kleiner 5, gemessen wie in der Beschreibung definiert, aufweist.

11. Polyglycerinpartialester nach mindestens einem der vorhergehenden Ansprüche, **dadurch gekennzeichnet, dass** er einen Schmelzpunkt von größer 35 °C, bevorzugt größer 40 °C, insbesondere größer 50 °C, besonders bevorzugt in einem Bereich von 51 °C bis 100 °C, gemessen wie in der Beschreibung definiert, aufweist.

12. Verfahren zur Herstellung eines Polyglycerinpartialesters umfassend die Verfahrensschritte

A) Bereitstellen eines Polyglycerins mit einem mittleren Kondensationsgrad N von 2,4 bis 15,0, bevorzugt 2,6 bis 10,0, insbesondere 2,8 bis 6,0,

B) Bereitstellen einer Carbonsäuremischung umfassend:

mindestens eine kurzkettige Dicarbonsäure mit 4 bis 18, bevorzugt 4 bis 14, besonders bevorzugt 6 bis 10, Kohlenstoffatomen und

mindestens eine langkettige, gesättigte und lineare Monocarbonsäure mit 18 bis 32, bevorzugt 18 bis 26, besonders bevorzugt 18 bis 22, Kohlenstoffatomen,

wobei die Summe aller in der Carbonsäuremischung enthaltenen kurzkettigen Dicarbonsäuren mindestens 75 Gew.-%, bevorzugt mindestens 85 Gew.-%, besonders bevorzugt mindestens 95 Gew.-%, bezogen auf alle in der Carbonsäuremischung enthaltenen Dicarbonsäuren, ausmacht,

wobei die Summe aller in der Carbonsäuremischung enthaltenen langkettigen, gesättigten und linearen Monocarbonsäuren mindestens 75 Gew.-%, bevorzugt mindestens 85 Gew.-%, besonders bevorzugt mindestens 95 Gew.-%, bezogen auf alle in der Carbonsäuremischung enthaltenen Monocarbonsäuren, ausmacht,

C) Verestern des Polyglycerins mit der Carbonsäuremischung, wobei das Molverhältnis aller in der Carbonsäuremischung enthaltenen Carboxylgruppen zu allen im eingesetzten Polyglycerin enthaltenen Hydroxylgruppen mindestens 50 zu 100, bevorzugt mindestens 55 zu 98, besonders bevorzugt mindestens 68 zu 95, beträgt,

**dadurch gekennzeichnet, dass** das Molverhältnis von Polyglycerin a) zu Dicarbonsäurekomponente b) zu Carbonsäurekomponente c) 1,0:1,0:2,1 bis 4,0:1,0:10,0, bevorzugt 1,0:1,0:3,1 bis 3,0:1,0:8,0, beträgt.

**13.** Formulierung, insbesondere eine kosmetische Formulierung, umfassend

1) Polyglycerinpartialester nach mindestens einem der Ansprüche 1 bis 11 oder erhältlich nach dem Verfahren des Anspruchs 12 und gegebenenfalls

2) mindestens eine Substanz ausgewählt aus der Gruppe umfassend UV-Lichtschutzfiltersubstanzen und Pigmente, insbesondere aus der Gruppe der UV-Lichtschutzfiltersubstanzen.

**14.** Formulierung nach Anspruch 13, **dadurch gekennzeichnet, dass** Komponente 2) ausgewählt ist aus der Gruppe der UV-Lichtschutzfiltersubstanzen umfassend Butyl Methoxydibenzoylmethane, Ethylhexyl Triazone, Bis-Ethylhexyloxyphenol Methoxyphenyl Triazine, Diethylhexyl Butamido Triazone, Diethylamino Hydroxybenzoyl Hexyl Benzoate, Benzophenone-3, Benzophenone-4, 4-Methylbenzylidene Camphor, Octocrylene, Ethylhexyl Methoxycinnamate, Ethylhexyl Salicylate, Homomenthyl Salicylate, Phenylbenzimidazol Sulfonic Acid, Methylene Bis-Benzotriazolyl Tetramethylbutylphenol, Disodium Phenyl Dibenzimidazole Tetrasulfonate, Isoamyl p-Methoxycinnamate und Ethylhexyl Dimethyl PABA und/oder ausgewählt ist aus der Gruppe der anorganischen Pigmente, insbesondere umfassend Aktivkohle, Talk, Eisenoxidpigmente, Titandioxid, Zinkoxid, Silica, Ceroxide, Zirconiumoxide, Aluminiumoxide, Calciumcarbonat, Bariumsulfat, Chromoxide, Manganoxide, Bismutoxychlorid, Ultramarin, Calciumsulfat und Alkalimagnesiumsilikate.

**15.** Formulierung nach Anspruch 13 oder 14, **dadurch gekennzeichnet, dass** Komponente 1) in einer Menge von 0,1 Gew.-% bis 20 Gew.-%, bevorzugt von 0,25 Gew.-% bis 12 Gew.-%, besonders bevorzugt von 0,5 Gew.-% bis 6 Gew.-%, vorliegt und Komponente 2) in einer Menge von 0,1 Gew.-% bis 60 Gew.-%, bevorzugt von 1 Gew.-% bis 50 Gew.-%, besonders bevorzugt von 3 Gew.-% bis 40 Gew.-%, vorliegt.

**16.** Verwendung eines Polyglycerinpartialesters nach mindestens einem der Ansprüche 1 bis 11 oder erhältlich nach dem Verfahren nach Anspruch 12 als Filmbildner, zur Steigerung des Lichtschutzfaktors von UV-Lichtschutzfiltersubstanzen oder zur Verringerung der Abwaschbarkeit und/oder des Abriebs einer Formulierung von einer Oberfläche.

**Revendications**

**1.** Ester partiel de polyglycérol pouvant être obtenu par estérification

a) d'un polyglycérol ayant un degré de condensation moyen N de 2,4 à 15,0, de préférence de 2,6 à 10,0, notamment de 2,8 à 6,0,

avec un mélange d'acides carboxyliques comprenant :

b) au moins un acide dicarboxylique à chaîne courte ayant 4 à 18, de préférence 4 à 14, plus préférablement 6 à 10, atomes de carbone et

c) au moins un acide monocarboxylique linéaire et saturé à longue chaîne ayant 18 à 32, de préférence 18 à 26, plus préférablement 18 à 22, atomes de carbone,

la somme de tous les acides dicarboxyliques à chaîne courte présents dans le mélange d'acides carboxyliques s'élevant à au moins 75 % en poids, de préférence au moins 85 % en poids, plus préférablement au moins 95 % en poids, sur la base de la totalité des acides dicarboxyliques présents dans le mélange d'acides carboxyliques, la somme de tous les acides monocarboxyliques saturés et linéaires à longue chaîne présents dans le mélange d'acides carboxyliques s'élevant à au moins 86 % en poids, de préférence au moins 90 % en poids, plus préférablement au moins 95 % en poids, sur la base de la totalité des acides monocarboxyliques présents dans le mélange d'acides carboxyliques,

le rapport molaire de tous les groupes carboxyle présents dans le mélange d'acides carboxyliques sur tous les groupes hydroxyle présents dans le polyglycérol utilisé étant d'au moins 50 à 100, de préférence d'au moins 55 à 98, plus préférablement d'au moins 68 à 95,

**caractérisé en ce que**

le rapport molaire du polyglycérol a) sur le composant acide dicarboxylique b) sur le composant acide monocarboxylique c) est de 1,0:1.0:2,1 à 4,0:1.0:10,0, de préférence de 1,0:1.0:3,1 à 3,0:1.0:8,0.

2. Ester partiel de polyglycérol selon la revendication 1, **caractérisé en ce que** le polyglycérol utilisé présente une teneur en glycérol de 0,05 % en poids à 25,0 % en poids, de préférence de 0,1 % en poids à 15,0 % en poids, plus préférablement de 0,1 % en poids à 10,0 % en poids.

3. Ester partiel de polyglycérol selon la revendication 1 ou 2, **caractérisé en ce que** le polyglycérol utilisé présente une teneur en diglycérol de 0,1 % en poids à 45,0 % en poids, de préférence de 0,5 % en poids à 35,0 % en poids, plus préférablement de 3,0 % en poids à 30,0 % en poids.

4. Ester partiel de polyglycérol selon au moins l'une des revendications précédentes, **caractérisé en ce que** le polyglycérol utilisé présente une teneur en isomères cycliques de 1 % en poids à 50 % en poids, de préférence de 2 % en poids à 40 % en poids, plus préférablement de 3 % en poids à 30 % en poids.

5. Ester partiel de polyglycérol selon au moins l'une des revendications précédentes, **caractérisé en ce que** l'acide dicarboxylique à chaîne courte est choisi parmi les acides dicarboxyliques aliphatiques linéaires, notamment l'acide oxalique, l'acide malonique, l'acide tartronique, l'acide succinique, l'acide maléique, l'acide tartrique, l'acide malique, l'acide fumarique, l'acide sorbique, l'acide $\alpha$-cétoglutarique, l'acide glutarique, l'acide adipique, l'acide pimélique, l'acide subérique, l'acide azélaïque, l'acide sébacique, l'acide undécanedioïque, l'acide dodécanedioïque et l'acide brassylique, avec une préférence particulière pour l'acide adipique, l'acide pimélique, l'acide subérique, l'acide azélaïque et l'acide sébacique.

6. Ester partiel de polyglycérol selon au moins l'une des revendications précédentes, **caractérisé en ce que** les acides monocarboxyliques linéaires et saturés à longue chaîne sont choisis parmi les acides gras substitués par hydroxy et non substitués, en particulier non substitués.

7. Ester partiel de polyglycérol selon au moins l'une des revendications précédentes, **caractérisé en ce que** la teneur totale en acides monocarboxyliques et dicarboxyliques aromatiques par rapport à tous les acides monocarboxyliques et dicarboxyliques utilisés est de 0,1 % en poids à 35 % en poids, de préférence de 0 % en poids à 25 % en poids, plus préférablement de 0 % en poids à 10 % en poids.

8. Ester partiel de polyglycérol selon au moins l'une des revendications précédentes, **caractérisé en ce qu'**il présente un degré d'estérification de 50 % à 100 %, de préférence de 55 % à 98 %, plus préférablement de 68 % à 95 %.

9. Ester partiel de polyglycérol selon au moins une des revendications précédentes, **caractérisé en ce qu'**il présente une valeur HLB de 2,0 à 8,0, préférablement de 2,5 à 6 et plus préférablement de 3 à 5,2, mesurée comme défini dans la description.

10. Ester partiel de polyglycérol selon au moins l'une des revendications précédentes, **caractérisé en ce qu'**il présente un indice d'iode inférieur à 20, de préférence inférieur à 12, particulièrement préférablement inférieur à 5, mesuré comme défini dans la description.

**11.** Ester partiel de polyglycérol selon au moins l'une des revendications précédentes, **caractérisé en ce qu'**il présente une température de fusion supérieure à 35 °C, de préférence supérieure à 40 °C, en particulier supérieure à 50 °C, plus préférablement comprise dans une plage de 51 °C à 100 °C, mesurée comme défini dans la description.

**12.** Procédé de préparation d'un ester partiel de polyglycérol, comprenant les étapes de procédé suivantes

A) fourniture d'un polyglycérol ayant un degré de condensation moyen N de 2,4 à 15,0, de préférence de 2,6 à 10,0, notamment de 2,8 à 6,0,
B) fourniture d'un mélange d'acides carboxyliques comprenant :

au moins un acide dicarboxylique à chaîne courte ayant 4 à 18, de préférence 4 à 14, plus préférablement 6 à 10, atomes de carbone et
au moins un acide monocarboxylique linéaire et saturé à longue chaîne, possédant 18 à 32, de préférence 18 à 26, plus préférablement 18 à 22, atomes de carbone,
la somme de tous les acides dicarboxyliques à chaîne courte présents dans le mélange d'acides carboxyliques s'élevant à au moins 75 % en poids, de préférence au moins 85 % en poids, plus préférablement au moins 95 % en poids, sur la base de la totalité des acides dicarboxyliques présents dans le mélange d'acides carboxyliques,
la somme de tous les acides monocarboxyliques saturés et linéaires à longue chaîne présents dans le mélange d'acides carboxyliques s'élevant à au moins 75 % en poids, de préférence au moins 85 % en poids, plus préférablement au moins 95 % en poids, sur la base de la totalité des acides monocarboxyliques présents dans le mélange d'acides carboxyliques,

C) estérification du polyglycérol par le mélange d'acides carboxyliques, le rapport molaire de tous les groupes carboxyle présents dans le mélange d'acides carboxyliques sur tous les groupes hydroxyle présents dans le polyglycérol utilisé étant d'au moins 50 à 100, de préférence d'au moins 55 à 98, plus préférablement d'au moins 68 à 95,

**caractérisé en ce que** le rapport molaire du polyglycérol a) sur composant acide dicarboxylique b) sur composant acide carboxylique c) est de 1,0:1.0:2,1 à 4,0:1.0:10,0, de préférence de 1,0:1.0:3,1 à 3,0:1.0:8,0.

**13.** Formulation, notamment formulation cosmétique, comprenant

1) des esters partiels de polyglycérol selon au moins une des revendications 1 à 11 ou pouvant être obtenus selon le procédé selon la revendication 12, et éventuellement
2) au moins une substance choisie dans le groupe comprenant les substances de filtre de protection contre de la lumière UV et les pigments, notamment dans le groupe des substances de filtre de protection contre de la lumière UV.

**14.** Formulation selon la revendication 13, **caractérisée en ce que** le composant 2)

est choisi dans le groupe des substances de filtre de protection contre de la lumière UV comprenant butyl methoxydibenzoylmethane, ethylhexyl triazone, bis-ethylhexyloxyphenol methoxyphenyl triazine, diethylhexyl butamido triazone, diethylamino hydroxybenzoyl hexyl benzoate, benzophenone-3, benzophenone-4, 4-methylbenzylidene camphor, octocrylene, ethylhexyl methoxycinnamate, ethylhexyl salicylate, homomenthyl salicylate, phenylbenzimidazole sulfonic acid, methylene bis-benzotriazolyl tetramethylbutylphenol, disodium phenyl dibenzimidazole tetrasulfonate, isoamyl p-methoxycinnamate et ethylhexyl dimethyl PABA et/ou
est choisi dans le groupe des pigments minéraux, comprenant notamment le carbone activé, le talc, des pigments d'oxyde de fer, le dioxyde de titane, l'oxyde de zinc, la silice, les oxydes de cérium, les oxydes de zirconium, les oxydes d'aluminium, le carbonate de calcium, le sulfate de baryum, les oxydes de chrome, les oxydes de manganèse, l'oxychlorure de bismuth, l'outremer, le sulfate de calcium et les silicates de magnésium et d'un métal alcalin.

**15.** Formulation selon la revendication 13 ou 14, **caractérisée en ce que**

le composant 1) est présent en une quantité de 0,1 % en poids à 20 % en poids, préférablement de 0,25 % en poids à 12 % en poids et plus préférablement de 0,5 % en poids à 6 % en poids, et
le composant 2) est présent en une quantité de 0,1 % en poids à 60 % en poids, préférablement de 1 % en poids à 50 % en poids et plus préférablement de 3 % en poids à 40 % en poids.

16. Utilisation d'un ester partiel de polyglycérol selon au moins l'une des revendications 1 à 11 ou pouvant être obtenu selon le procédé selon la revendication 12 comme agent filmogène, pour augmenter le facteur de protection solaire de substances de filtre de protection contre de la lumière UV ou pour réduire la rinçabilité et/ou l'usure d'une formulation à partir d'une surface.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0835862 A
- EP 1500427 A
- EP 1683781 A
- EP 3500550 A
- EP 2593076 A
- EP 2363387 A
- EP 1417955 A
- WO 2015013951 A
- DE 102008001788

**Non-patent literature cited in the description**

- **CASSEL et al.** Original synthesis of linear, branched and cyclic oligoglycerol standards. *J. Org. Chem.*, 2001, 875-896
- **P. FINKEL**. *SOFW Journal*, 1996, vol. 122, 543
- The Chemistry of Ultraviolet Filters. **N.A. SHAATH**. Principles and Practice of Photoprotection. Springer International Publishing, 2016
- Kosmetische Färbemittel. Cosmetic colorants] of the Dyes Committee of the Deutsche Forschungsgemeinschaft [German Research Foundation. Verlag Chemie, 1984, 81-106
- Grundlagen und Rezepturen der Kosmetika. **K. SCHRADER**. Fundamentals and formulations of cosmetics. Hüthig Buch Verlag, 329-341